# EUROPEAN PATENT APPLICATION

(11) **EP 3 578 653 A2**
(43) Date of publication of application: **11.12.2019**
(21) Application number: 19177160.9
(22) Date of filing: 29.05.2019
(51) Int. Cl.: C12N 15/10, C12N 15/115

(54) **METHOD OF APTAMER SELECTION AND METHOD FOR PURIFYING BIOMOLECULES WITH APTAMERS**

(30) Priority: 04.06.2018 CN 201810563602
(71) Applicant: Hong, Chin-Yih, Changhua 51065 (TW); Horng, Herng-Er, New Taipei City 244 (TW)
(72) Inventor: Hong, Chin-Yih, Changhua 51065 (TW); Horng, Herng-Er, New Taipei City 244 (TW)
(74) Representative: Becker, Eberhard

(57) **Abstract**

The present invention proposes a method of using oligonucleotide aptamers binding with certain specific biomolecules associated with a disease to purify the specific biomolecules from disease patient's samples and to identify biomarkers for the particular disease. The present invention describes a method using the magnetic assisted rapid aptamer selection (MARAS) to select aptamers having high binding affinity for certain disease-related biomolecules and not for other non-disease-related biomolecules from nucleic acid libraries. Meanwhile, the present invention also proposes a method of using the aptamer obtained above as a capture ligand to purify certain specific biomolecules related to a disease from disease patients' samples. Further, the present invention describes the use of the obtained aptamer as the capture ligand to purify biomolecules with a specific binding affinity range by applying an oscillating magnetic field range as a virtual filter.

SEQUENCE LISTING

<110>  Hong, Chin-Yih

<120>  METHOD OF APTAMER SELECTION AND METHOD FOR PURIFYING BIOMOLECULES
        WITH APTAMERS

<130>  77216-EP-PA

<150>  CN 201810563602.6
<151>  2018-06-04

<160>  7

<170>  PatentIn version 3.5

<210>  1
<211>  60
<212>  DNA
<213>  Artificial Sequence

<220>
<223>  nucleic acid library


<220>
<221>  misc_feature
<222>  (21)..(40)
<223>  n is a, c, g, or t

<400>  1
agcagcacag aggtcagatg nnnnnnnnnn nnnnnnnnnn cctatgcgtg ctaccgtgaa      60


<210>  2
<211>  20
<212>  DNA
<213>  Artificial Sequence

<220>
<223>  label forward

<400>  2
agcagcacag aggtcagatg                                                  20


<210>  3
<211>  20
<212>  DNA
<213>  Artificial Sequence

<220>
<223>  label reverse

<400>  3
ttcacggtag cacgcatagg                                                  20


<210>  4
<211>  20
<212>  DNA
<213>  Artificial Sequence

<220>
<223>  universal T7 primer

<400>  4
taatacgact cactataggg                                                   20


<210>  5
<211>  20
<212>  DNA
<213>  Artificial Sequence

<220>
<223>  aptamer

<400>  5
ggccctccag ccatgctgtg                                                   20


<210>  6
<211>  20
<212>  DNA
<213>  Artificial Sequence

<220>
<223>  aptamer

<400>  6
ggtggcagcg taagggcaat                                                   20


<210>  7
<211>  20
<212>  DNA
<213>  Artificial Sequence

<220>
<223>  aptamer

<400>  7
gtctcggccc accctcgcga                                                   20

## Description

### BACKGROUND

### Technical Field

The present invention generally relates to methods of the use of a design with positive and negative selections and the structural diversity in nucleic acid libraries to find the difference between positive samples (samples of disease patients) and negative samples (samples of non-disease patients), the use of the magnetic assisted rapid aptamer selection (MARAS) method to select aptamers having the ability to distinguish between these two, and the use of the selected aptamers as capture ligands to purify some particular disease-related biomolecules bound to the aptamers from positive attribute samples, which are to be identified in order to find the disease-related biomarkers. In particular, the present invention relates to a selection method of aptamers from a nucleic acid library, which have the ability to distinguish the difference between disease patients' and non-disease patients' samples and can be used as capture ligands to purify this difference, the disease-related biomolecules from disease patients' samples. Meanwhile, the present invention describes a method of the use of this aptamer as a capture ligand to purify this difference, disease-related biomolecules from disease patients' samples.

### Description of Related Art

Purification of a target protein from a total protein and identification of the target protein must rely on appropriate protein purification techniques to separate the target protein from the total protein for subsequent analysis. Common protein purification techniques are: (1) Membrane Separation uses the molecular size of the protein to achieve purpose of separation in most processes; (2) Chromatography uses the molecular size, charge, polarity of the protein, and the different affinity and is divided into Gel Filtration, Ion-Exchange Chromatography, Hydrophobic Interaction Chromatography (HIC), Affinity Chromatography, Partition Chromatography, and High-Performance (Pressure) Liquid Chromatography; and (3) Magnetic Separation utilizes its own magnetic properties. A molecule (DNA, antibody, or peptide) capable of specifically binding to a target protein is immobilized on the surface of the magnetic particle and binds to the target protein. The target protein can be quickly obtained from the total protein by performing a magnetic separation using a magnet and the effect of separation can be achieved. When the molecular weight of the target protein is close to that of the non-target protein, the purification methods of (1) and (2) above cause the target protein to be mixed with the non-target protein, resulting in a decrease in purity, such as a membrane separation is used as a method for preliminary purification of proteins to be separated; and (3) magnetic separation method can quickly separate the target protein from total protein, but in order to desorb the target protein from the magnetic beads, it is usually washed with a high concentration of salt (elution). The elution step is time consuming and may cause a decrease in protein activity. Furthermore, the magnetic separation method often requires an antibody as a tool for binding to a protein. The antibody is expensive, the storage is not easy, and the potency is different, resulting in an excessively high protein purification cost. Therefore, it is not easy to be promoted for practical use.

It is used the methods such as enzyme-linked immunosorbent assay (ELISA), quantitative immuno-PCR (QI-PCR), protein microarray, two-dimensional gel electrophoresis, mass spectrometry, shut gun proteomics, isotope-code affinity tagging combined with mass spectrometry (ICAT) or image analysis medium-assisted laser desorption/ionization-image mass spectrometry (MALDI-IMS), oligonucleotide aptamers, and so on to discover cancer-related biomarkers. All of the above experimental methods have high-flux characteristics, which can analyze the difference in protein or RNA expression between tumor patients and non-tumor normal people. However, the above experimental methods require professional techniques, large amount of labor, time, consumables, and expensive operations of instrument analysis. However, when a suitable monoclonal antibody with high affinity and specificity does not exist or the identity of target biomolecule is unknown, the above method cannot achieve the purpose of protein purification and thus the biomarker cannot be identified.

By utilizing the diversity of the constituent oligonucleotides in the nucleic acid library to generate the diversity of the structures formed by folding the oligonucleotides which bind to the target biomolecule, a competitive mechanism is applied to select aptamers of high binding affinity and high specificity to the target biomolecule from the nucleic acid library. The aptamers above can be used to replace monoclonal antibodies. On the other hand, if the identity of the target biomolecule is not known, the diversity of the structures formed by folding the oligonucleotides in the nucleic acid library can still be utilized to select aptamers capable of distinguishing the samples with different attributes (disease patients and non-disease patients).

Aptamers including binding pockets bind with high specificity and affinity to a variety of target analytes, diversified from micro-molecules (such as organic molecules, ions, peptides, proteins, and nucleic acids), macro-molecules to even whole cells, viruses, parasites or tissues. Once the sequence of aptamer is identified for the target analytes, the entire aptamer can be produced by chemical synthesis. Furthermore, aptamers modified with functional groups can increase their stability in various biological applications, but may be harmful for oligonucleotides (aptamers). Aptamers not only have the potential to be an excellent tool to target pathogenic malignant cells or tissues and substitute antibodies but also can be applied on purification, diagnostics, biosensors and anti-infectious agents. As the aptamers are potential in many aspects, an efficient selection method, Magnetic-Assisted Rapid Aptamer Selection (MARAS), has been developed which is straightforward enough to rapidly select suitable aptamers with high affinity and specificity for their target analytes. The detail description of MARAS given in the Chinese patent (ZL 2014 1 0570602.0) issued on March 8, 2017, which is the same as the former European patent (patent no. 2865753) issued on March 29, 2017, is hereby incorporated by reference herein and made a part of this specification.

MARAS does not require a selection cycle, and is a direct selection method that is simple, rapid, efficient, and is a development platform ensuring a high binding affinity and specificity of the aptamer toward target that can increase the scope of aptamer application. The method is to incubate magnetic particles conjugated with target molecules with a nucleic acid library, and a part of the oligonucleotides associates with the target molecule to form magnetic particle bound complexes. The magnetic particle bound complex in an aqueous solution moves under the action of an oscillating magnetic field of the MARAS through a magnetic traction force generated on the magnetic particle by the magnetic field that results an oscillating motion of the magnetic particle bound complex. When the magnetic particle bound complex moves in an aqueous solution, a viscous force opposite to the direction of motion is developed. Under the interaction of these two forces, the bonds on the target molecule to the magnetic particle and the aptamer to the target molecule undergo stretch forces that provide the competitive mechanism for selecting the aptamers. The competition mechanism of selection selects aptamers with high binding affinity and specificity for the target molecule. At the same time, the binding affinity (or equilibrium dissociation constant) between the aptamer and the target molecule can be chosen by using the conditions of the applied oscillating magnetic field to obtain an aptamer having a desired binding affinity for the target molecule.

### SUMMARY

Some aspects of the invention relate to the selection method using MARAS to select aptamers that have the ability to distinguish between patients' samples and non-patients' samples. This method utilizes the structural diversity formed by folding the oligonucleotides of the nucleic acid library and selects aptamers, which bind to biomolecules associated with a certain disease in positive (disease patient) samples and do not bind to non-disease-related biomolecules in positive and negative (non-disease patient) samples, from the nucleic acid library. Furthermore, the obtained aptamers are used to distinguish between positive and negative samples.

The present invention provides an aptamer selection method utilizing bio-functionalized magnetic particles to select aptamers, having the ability to distinguish the positive and negative samples, from the nucleic acid library.

Some aspects of the invention relate to methods of using the obtained aptamers, having the ability to distinguish positive and negative samples, as capture ligands to purify certain disease-related biomolecules from the positive attribute samples.

The present invention provides a method for purifying certain disease-related biomolecules from positive attribute samples, that uses aptamers as capture ligands conjugated to bio-functionalized magnetic particles to form purification reagents for certain disease-related biomolecules to purify these disease-related biomolecules from positive attribute samples.

In order to make the aforementioned and other features and advantages of the disclosure comprehensible, an exemplary embodiment accompanied with figures is described in detail below.

### BRIEF DESCRIPTION OF THE DRAWINGS

The accompanying drawings are included to provide a further understanding of the disclosure, and are incorporated in and constitute a part of this specification. The drawings illustrate the embodiment of the disclosure and, together with the description, serve to explain the principles of the disclosure.
FIG. 1 schematically illustrates, in accordance with the technique of the present invention, a selection method for selecting aptamers, having the ability to distinguish between disease-patients' and non-disease-patients' samples, using the MARAS method.
FIG. 2 schematically illustrates, in accordance with the technique of the present invention, a purification method that uses aptamers, having the ability to distinguish between positive samples and negative samples, as capture ligands, couple with a virtual filter simulated by an oscillating magnetic field range, for purifying certain disease-related biomolecules from positive attribute samples.
FIG. 3A is a schematic diagram of the experimental setting for the rotating magnetic field magnetic-assisted rapid aptamer selection (RO-MARAS) method according to the embodiment of the present invention.
FIG. 3B is a schematic diagram of the experimental setting for the alternating magnetic field magnetic-assisted rapid aptamer selection (AC-MARAS) method according to the embodiment of the present invention.
FIG. 4A-4C show the results of reverse validation of three obtained aptamers capable of distinguishing positive samples and negative samples of EGFR-mutated non-small cell lung cancer (NSCLC) disease patients via quantitative real-time PCR (q-PCR) according to the embodiment of the present invention.
FIG. 5A-5C show the results of blind sample analysis, using several blind test samples, of three obtained aptamers capable of distinguishing positive samples and negative samples of EGFR-mutated NSCLC disease patients via q-PCR according to the embodiment of the present invention.
FIG. 6 shows a blotting image of polyacrylamide gel electrophoresis of certain unknown analytes purified from positive attribute blind test samples of EGFR-mutated NSCLC patients using Aptamer37 as a capture ligand according to the embodiment of the present invention.
FIG. 7A-7C show the spectra of three biomolecule bands of the gel blot by MALDI-TOF mass spectrometer of unknown analytes purified from positive attribute blind test samples of EGFR-mutated NSCLC patients by Aptamer37 as a capture ligand according to the embodiment of the present invention.

### DESCRIPTION OF THE EMBODIMENTS

The content of the present invention mainly includes two parts, namely, selecting an aptamer, having the ability to distinguish between positive samples (disease patients' samples) and negative samples (non-disease patients' sample), from a nucleic acid library by a MARAS method, and purifying disease-related biomarkers from positive attribute samples using the obtained aptamers as capture ligands. The following is a detailed description of these two parts.

Some aspects of the present invention relate to the use of a diversity of constituent oligonucleotides in a nucleic acid library to produce a structural diversity for selecting aptamers, having an ability to distinguish positive samples (disease patients' samples) and negative samples (non-disease patients' samples), using MARAS method from the nucleic acid library. Through the design and implementation of positive selection and negative selection, the obtained aptamers only bind to biomolecules related to certain diseases in the positive samples, but not biomolecules unrelated to the disease in positive and negative samples. Furthermore, the aptamers, having the ability to distinguish the positive samples and negative sample, are selected from the nucleic acid library. At the same time, in order to verify the aptamer selection process, the obtained aptamers are used as detection probes for reverse verification of the positive and negative samples used in the selection process to verify the correctness of the process. Moreover, in order to validate the applicability in clinical diagnoses, the obtained aptamers are used as detection probes for a blind sample analysis using blind test samples which have existing pathological data (samples not used for positive or negative selection). The blind sample analysis is performed and the results are compared with the known pathological data, and the ability of the obtained aptamer to distinguish the positive blind test samples and the negative blind test samples is examined.

Further aspects of the invention relate to a method that uses an aptamer, having the ability to distinguish positive and negative samples, as a capture ligand in a purification reagent, using virtual filters simulated by manipulating the applied oscillating magnetic field ranges, to purify some disease-related biomolecules from positive attribute samples and identify the purified biomolecules to confirm the biomarkers related to the disease. Since the biomolecules purified during the purification process cannot be known prior to purification, the binding affinities (or equilibrium dissociation constants) between these biomolecules and the obtained aptamers cannot be measured in advance. Moreover, during the purification process of the biomolecules and the selection process of the aptamers, the architectures of the magnetic particle bound complex are different, i.e., target-aptamer-magnetic particle versus aptamer-target-magnetic particle, respectively. Therefore, the stretch forces, acting on the bond between the aptamer and the biomolecule, induced by the same oscillating magnetic field of MARAS, are different due to the difference on the outmost component of the complex (target versus aptamer). So, the oscillating magnetic field conditions that should be used for purification cannot be known prior to the purification. However, the binding affinity between the aptamer obtained by MARAS selection and the target biomolecules is a function of the condition of the applied oscillating magnetic field, and the greater the frequency and/or strength of the magnetic field is applied, the higher the binding affinity (the lower the equilibrium dissociation constant) among the obtained aptamers and the target biomolecules is achieved. The MARAS therefore provides a mechanism for the binding affinity selection of the joining pair formed by the aptamers and the target biomolecules. Another aspect of the present invention discloses a technique for purifying unknown biomolecules from samples by utilizing the characteristics of the binding affinity selection ability of MARAS for a joining pair formed by an aptamer and a target biomolecule and manipulating the frequency/strength range of the oscillating magnetic field to purify biomolecules from the positive attribute samples, of which the biomolecules purified by using different frequency/strength range of oscillating magnetic field have different binding affinity ranges toward the obtained aptamers, thereby purifying certain disease-related biomolecules from the positive attribute samples.

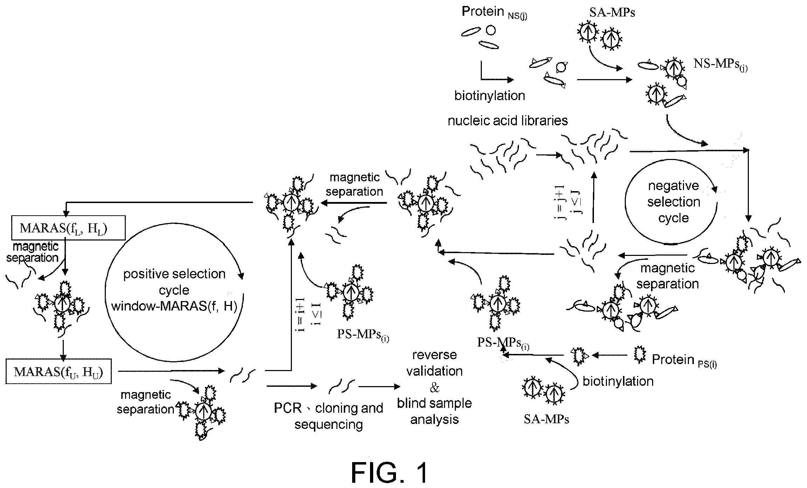

Before demonstrating the purification of certain disease-related biomolecules from disease patients' samples (positive attribute samples), aptamers capable of distinguishing the disease patients' samples (positive selection samples) and non-disease patients' samples (negative selection samples) must be selected from a nucleic acid library for serving as capture ligands for purification. In the present invention, the aptamer obtained by MARAS is further used as a capture ligand to purify certain disease-related biomolecules from disease patients' samples (positive attribute samples) to verify the feasibility of the present invention. As shown in Figure 1, a procedure of a selection (or generating) method for aptamers, having the above characteristics, is schematically illustrated. In the procedure depicted in Figure 1, four main parts are involved: (1) material preparation; (2) negative selection; (3) positive selection; and (4) post-analysis, including PCR (polymerase chain reaction) amplification, cloning, sequencing, reverse validation, and blind sample analysis. The details of the method or steps of four main parts are described below.

In the process of material preparation, J types of non-disease patients' samples are provided as negative selection samples (negative samples), I types of disease patients' samples are provided as positive selection samples (positive samples), and K types of patients' samples (including samples of disease patients and non-disease patients) are provided as samples for blind sample analysis (blind test samples). These samples are individually biotinylated and subsequently bonded to streptavidin-coated magnetic particles (SA-MPs) to form negative sample-magnetic particles (NS-MPs₍ⱼ₎), positive sample-magnetic particles (PS-MPs₍ᵢ₎), and blind test sample-magnetic particles (BS-MPs₍ₖ₎). The subscripts j, i, and k of NS-MPs, PS-MPs, and BS-MPs are independently positive integers starting from 1 to J, I, and K, respectively. It should be noted that in the entire embodiment including the selection processes of aptamer generation and the processes of reverse verification, blind sample analysis, and biomolecule purification using the aptamer-based reagent, the magnetic particles (MPs) used are not limited to magnetic nanoparticles (MNPs). The use of magnetic micro particles (MMPs) achieves similar results in the following experiments similar to those in the experiments of the MARAS procedure. The nucleic acid library includes randomized oligonucleotides flanked with primers at both ends for PCR amplification. A set of primers, marked as Lab-forward primer and Lab-reverse primer, is used for annealing the resulting oligonucleotide degenerating region during PCR amplification. A universal T7 primer is used to sequence the selected aptamer.

For the negative selection, the nucleic acid library is first dissolved in phosphate buffer (Phosphate Buffer Saline, PBS, pH 7.4), heated to 95°C (5 minutes), rapidly cooled to 4°C (2 minutes), and then placed at room temperature (60 minutes), for the oligonucleotides to form specific secondary or tertiary structures. After that, the nucleic acid library is incubated with NS-MPs₍₁₎. After incubation, a magnetic separation is performed to remove the bound mixture and collect the supernatant containing the remaining oligonucleotides that do not bind to NS-MPs(i). The collected supernatant is then incubated with the next NS-MPs₍₂₎ and the process is repeated until all J negative selections using J types of NS-MPs₍ⱼ₎, respectively, are completed. The purpose of multiple rounds of negative selection is to consider the composition of the individual negative samples to be different, so that the possibility of binding between the selected aptamers and the biomolecules in all J types of negative samples is minimized in order to increase the ability of selected aptamers to distinguish disease patients' samples and non-disease patients' samples. The same principle can be applied to the selection of aptamers for immunoassays to enhance detection sensitivity, and the aptamers selected for the immunoassays can only conjugate to the analyte of interest to reduce possible false-positive detection during immunoassay applications. In theory, the more negative selection operations are performed (the larger J), the higher the sensitivity that can be achieved during the immunoassays (the fewer false-positive detections) and the higher the discriminating rate for disease patients and non-disease patients. The final supernatant after the negative selection is collected and used for the following positive selection. Alternatively, one negative selection operation using a mixed NS-MPs from all J types of NS-MPs₍ⱼ₎ instead of multiple rounds of negative selection using individual NS-MPs₍ⱼ₎ will achieve the same result. However, it must be noted that with mixed NS-MPs, the concentration of MPs in PBS buffer may become too high and MPs become easy to agglomerate that adversely affects the selection. On the other hand, in biological systems, specific biomolecules may present in the samples of both disease and non-disease patients, but only at different concentrations. If the concentration of a specific biomolecule contained in the samples of non-disease patients is much lower than that contained in the samples of the disease patients, the specific biomolecule can also play the role of a biomarker for diagnosing the disease. In this case, the negative sample contains these specific biomolecules, and the negative selection process in the aptamer selection will exclude some of the oligonucleotides that may bind to these specific biomolecules, thereby reducing the efficiency of selection. However, the negative selection of the present invention does not affect the yield of the final aptamers selected, as the concentration of the specific biomolecule is very low in the negative samples and the total amount of oligonucleotides contained in the nucleic acid library used is large.

For the positive selection, the supernatant collected from the negative selection is incubated with PS-MPs₍₁₎ in PBS buffer. After incubation, a magnetic separation is performed to collect the bound mixture while discarding the supernatant containing unbound oligonucleotides. The collected bound mixture is dispersed in PBS buffer. The bound mixture solution is subjected to MARAS under a first oscillating magnetic field having a lower cutoff frequency f_{L} and/or a lower cutoff strength H_{L}, and a portion of oligonucleotides having a binding affinity to the PS-MPs₍₁₎ less than the stretch force induced by the first oscillating magnetic field detaches from the bound mixture. The supernatant containing the detached oligonucleotides is removed by magnetic separation, and the collected bound mixture is re-dispersed in PBS buffer. Next, a second oscillating magnetic field having an upper cutoff frequency fu and/or an upper cutoff strength Hu is applied to the bound mixture solution to detach oligonucleotides which bind to PS-MPs(i) with a desired binding affinity range to the joining pair formed by the oligonucleotides and biomolecules of PS-MPs₍₁₎, wherein the desired range has a binding affinity greater than the stretch force induced by the first oscillating magnetic field and less than the stretch force induced by the second oscillating magnetic field. A magnetic separation is performed to remove the bound mixture and collect the supernatant containing the oligonucleotides having the desired binding affinity range for PS-MPs₍₁₎. The supernatant is then incubated with the next positive sample (PS-MPs₍₂₎) and the procedure is repeated until all I positive selections using I types of PS-MPs₍ᵢ₎, respectively, are completed. The oligonucleotides contained in the final supernatant are the desired aptamers. The purpose of multiple rounds of positive selection is to consider the differences in the composition of individual positive samples, so that the aptamers obtained can only bind to the common spices of biomolecules contained in all of the I types of positive samples to increase the discriminating ability of patients' samples of the obtained aptamers, which in turn reduce the false-negatives at the time of discrimination. The aptamer-containing supernatant after the positive selection procedure is then collected for the following post-analysis, such as PCR amplification, cloning, sequencing, reverse validation, and blind sample analysis for blind test samples. The procedure for generating an aptamer having the ability to distinguish disease patients' samples (positive selection samples) and non-disease patients' samples (negative selection samples), including negative and positive selections, is schematically illustrated in Figure 1. In order to achieve the above purpose, during the application of the oscillating magnetic field, a constraint is imposed on the frequency (f) and the strength (H) of the applied oscillating magnetic field, that is, f_{L} ≤ f_{U} and/or H_{L} ≤ H_{U}, and wherein the equality cannot co-exist, i.e., f_{L} = f_{U} and H_{L} = H_{U} cannot be applied simultaneously. The oscillating magnetic field used can be a rotating, alternating, or elliptical magnetic fields. In addition, it is worth mentioning that for each round of positive selection, the oscillating magnetic fields (f_{L}, H_{L}, f_{U} and H_{U}) used are not strictly required to be the same, and only the oscillating magnetic fields that yield the aptamers obtained with high binding affinity to PS-MPs₍ᵢ₎ are sufficient. The above-mentioned Chinese invention patent (ZL 2014 1 0570602.0) provides a reference for choosing the range of the oscillating magnetic field according to the range of binding affinity (equilibrium dissociation constant) required and the details of the post analysis including PCR amplification, cloning and sequencing. Alternatively, in each round of positive selection, after applying a first oscillating magnetic field having a lower cutoff frequency f_{L} and/or a lower cutoff strength H_{L}, the collected bound mixture is re-dispersed, heated, eluted, magnetically separated and purified to obtain the supernatant containing the oligonucleotides for the next round of positive selection or post-analysis. And, the binding affinity of the joining pairs formed by the aptamers obtained to the biomolecules is greater than the stretch force induced by the first oscillating magnetic field. The aptamers obtained by selection are then subjected to PCR amplification, cloning and sequencing. Also, these aptamers are used as detection probes for reverse validation using negative selection samples and positive selection samples. In addition, similar to that in the MARAS procedure, the negative selection followed by the positive selection or the positive selection followed by the negative selection, the selecting results of the aptamers are similar. In the technique disclosed by the present invention, the order of the positive and negative selections does not affect the results of selecting aptamers. Once the aptamers obtained have been validated (reverse validation), they can be used as detection probes for blind sample analysis or as capture ligands of the purification reagent to purify specific disease-related biomarkers from positive attribute samples.

It is also worth mentioning that if the composition of the negative sample is fixed, only one sample (J = 1) is required for the negative selection, and only one sample (I = 1) is required for the positive selection if the target biomolecule is fixed. For example, when the former wants to identify or purify a specific tumor cell-related metabolite from a tumor cell culture solution, the normal cell culture solution can be used as a negative sample. Since the culture solution is generated in a controlled environment and the composition thereof is fixed, so only one single negative selection sample is needed. The latter is used to select aptamers with high specificity and binding affinity to a specific target biomolecule (target) to be used as detection probes for immunoassays or as capture ligands for purification of the biomolecule with known identity from samples. Since the target or target biomolecule is fixed and known, only the known target or the target biomolecule is needed to be used as a positive sample for positive selection, so only a single positive selection is required.

In addition, the competition mechanism provided by MARAS for aptamer selection is based on the application of stretch force to the joining pair formed by the aptamer and the biomolecule, so any method or facility that can exert a stretch force on the joining pair can be used to resemble MARAS that provides a competitive mechanism for aptamer selection, including but not limited to mechanical forces (e.g., in the case of motions of bound complexes consisting of magnetic particles and aptamer-biomolecules driven by oscillating magnetic fields in an aqueous solution; in the case of aptamer-biomolecule complex attached to a fixed substrate or captured (e.g., a fluid channel), the fluid dynamic force induced by the rigorous washing; and in the case of aptamer-biomolecule complex attached to a fixed substrate or captured (e.g., fluid channel) in a lab-on-a-disc, the centrifugal force induced by a rotation of aptamer-biomolecule complex, etc.), electromagnetic forces (e.g., the static magnetic force induced by a gradient magnetic field in the case of using a magnetic substance and the static electric force induced by electric field in the case of using a charged substance), or any combination of the above forces.

The reverse validation and blind sample analysis can be performed by techniques commonly used in general medical diagnoses such as ELISA, real-time quantitative PCR (q-PCR) or nephelometry. The following experiments briefly describe the steps of using the aptamer obtained by MARAS as a detection probe for reverse validation and blind sample analysis by the q-PCR method. First, overdosed aptamers are heated and rapidly cooled in PBS buffer as described above to form specific secondary or tertiary structures, and incubated individually with the reverse validation sample-magnetic particles (J types of NS-MPs₍ⱼ₎ and I types of PS-MPs₍ᵢ₎) and blind test sample-magnetic particles (K types of BS-MPs₍ₖ₎), followed by magnetic separation. The supernatant containing unbound aptamers is removed and the magnetic particle bound complex is retained. The bound complex is washed with PBS buffer to completely eliminate nonspecific binding, then dispersed and heated with deionized water to elute the aptamers bound to the magnetic particle bound complex. A magnetic separation is performed to remove the magnetic particles and the supernatant containing the eluted aptamers is retained. Finally, the amount of the aptamer contained in the supernatant is measured by q-PCR in duplex and presented as a relative expression. It is worth mentioning that in the blind sample analysis, for minimizing the possibility of nonspecific binding, the first oscillating magnetic field condition (the lower cutoff frequency f_{L} and/or the lower cutoff strength H_{L}) used for the aptamer selection can be applied after the incubation in order that the aptamers having the binding affinity to the biomolecules less than the stretch force induced by the first oscillating magnetic field to the joining pair formed by the aptamer and the biomolecule are detached from the magnetic particle bound complex and eliminated through the magnetic separation for further ruling out nonspecific bindings.

In the present invention, the purification procedure of using the aptamer obtained by the aforementioned procedure as a capture ligand to purify certain disease-related biomolecules from disease patients' samples (positive attribute samples) is performed by applying an oscillating magnetic field to the magnetic particle bound complex consisting of magnetic particle conjugated with aptamers as capture ligands binding to certain specific biomolecules associated with a disease, to generate a stretch force against joining pairs formed by aptamers and biomolecules, thereby causing the specific biomolecules having a specific binding affinity range to detach from the magnetic particle bound complex, and the supernatant is collected by magnetic separation to obtain the specific biomolecules having a specific binding affinity range to the aptamers. Because the architecture of the magnetic particle bound complex (target-aptamer-magnetic particle) during the purification process and that (aptamer-target-magnetic particle) during aptamer selection process are different, therefore, under the same oscillating magnetic field, the evoked stretch forces (defined by the viscous force opposite to the direction of motion of the magnetic particle bound complex in an aqueous solution) on the joining pair formed by aptamers and biomolecules are different due to the different components of the outermost layers of the bound complexes (target versus aptamer). Thus, the oscillating magnetic field conditions that should be used for purification cannot be known prior to purification. Theoretically, the larger the component of the outermost layer of the bound complex is, the greater the stretch force is induced by the same oscillating magnetic field on the joining pair formed by the aptamer and the biomolecule. Generally, the size of the biomolecule to be purified is larger than the aptamer, so the stretch force induced by the same oscillating magnetic field on the joining pair formed by the aptamer and the biomolecule during the purification is greater than that during aptamer selection. On the other hand, the binding affinity of the aptamer to the biomolecule to be purified is fixed. It is thus expected that the oscillating magnetic field condition required to detach the biomolecule to be purified from the aptamer during purification is lower than the oscillating magnetic field condition that causes the aptamer to detach from the biomolecule during aptamer selection. Therefore, in the present invention, a scanning method of oscillating magnetic field condition, that is, a scanning method of binding affinity, can be performed to obtain and purify disease-related biomolecules having different binding affinity ranges to the aptamer. On the other hand, since the amount of the sample (positive attribute sample) of a patient with a specific disease may be limited, the amount of the biomolecule purified according to the above steps may be not sufficient to be sequenced by a mass spectrometer to confirm the identity. Therefore, the same oscillating magnetic field conditions can be applied to samples of different patients with the same disease (different positive attribute samples) to perform a binding affinity range scan, and the purified biomolecules from multiple disease patients' samples having the same binding affinity range to the aptamer are collected for the mass spectrometry analysis to confirm the identity.

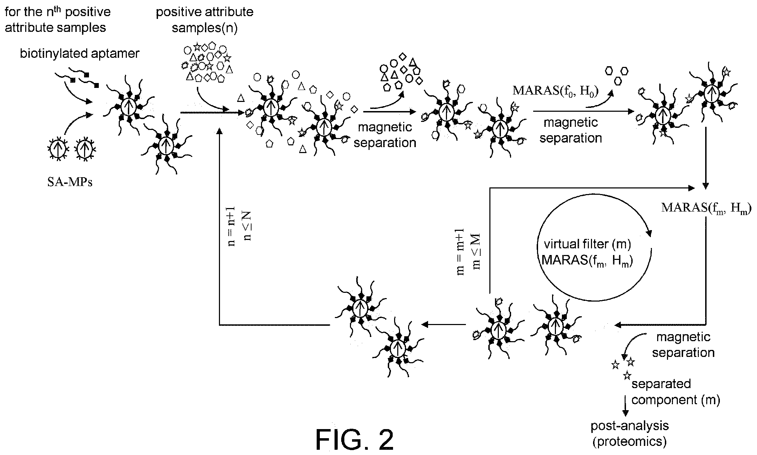

The purification reagent must be prepared prior to purification. For the preparation of the purification reagent, the previously validated aptamer is used as a capture ligand for the purification reagent. An excess amount of biotinylated aptamer is dissolved in PBS buffer, heated, and rapidly cooled as described above for forming specific secondary or tertiary structures. The aptamer solution is incubated with SA-MPs, and magnetically separated to remove the supernatant containing the aptamers not bound to the magnetic particles and retain the magnetic particle bound complex. The magnetic particle bound complex is dispersed in PBS buffer as a purification reagent. After the preparation of purification reagent, it can be used to purify the unknown biomolecules related to disease from blind test samples with a high relative expression level to the aptamer during the blind sample analysis by q-PCR. A flow chart for the purification of unknown biomolecules related to disease in the samples is shown in Figure 2.

Figure 2 illustrates that the N samples (positive attribute samples) with high relative expression level of q-PCR results in the blind sample analysis for the aptamer are first chosen and used as positive attribute samples to be purified, followed by incubating the positive attribute sample (1) with the purification reagent. A magnetic separation is performed to remove the supernatant containing the portion not bound to the magnetic particles conjugated with the aptamer in the purification reagent and collect the magnetic particle bound complex. The collected bound complex is washed several times and dispersed with PBS buffer. And secondly, an initial (0^{th}) oscillating magnetic field having a frequency f₀ and a strength H₀ is applied to the dispersed solution of the magnetic particle bound complex to detach the portion of biomolecules having binding affinity lower than the initial (0^{th}) binding affinity, that is, binding to the aptamer nonspecifically or with low binding affinity. The supernatant containing the detached portion is again removed by magnetic separation, and the magnetic particle bound complex is collected, washed several times, and dispersed with PBS buffer, wherein the initial binding affinity is defined by the stretch force induced by the initial oscillating magnetic field on the joining pair formed by the aptamer and biomolecule. Again, a first oscillating magnetic field having a frequency f₁ and/or a strength H₁ is applied to the dispersed solution of the magnetic particle bound complex, so that the portion of the biomolecules having a binding affinity between the initial and the first binding affinity to the aptamer detaches from the magnetic particle bound complex and a magnetic separation is performed to collect the supernatant containing the separated fraction (separated component (1)), while collecting and dispersing the magnetic particle bound complex in PBS buffer, and applying a second oscillating magnetic field with frequency f₂ and/or strength H₂, and repeating the purification procedure for the positive attribute sample (1) until purification and collection of all M separated components are completed, wherein the first binding affinity is defined by the stretch force on the joining pair formed by the aptamer and biomolecule induced by the first oscillating magnetic field. The range of binding affinity defined by the initial and first oscillating magnetic fields can be regarded as a virtual filter (1) for selecting biomolecules that have a binding affinity to the aptamer within the above range. In other words, for the purification procedure of the unknown biomolecules of any positive attribute sample (n), a total of M rounds are performed, that is, M virtual filters are used. After the final round of purification and collection of the separated component (M) of the positive attribute sample (1), the magnetic particle bound complex is dispersed in PBS buffer and used as a purification reagent or a purification reagent is re-prepared according to the above preparation method for the next positive attribute sample. Next, the positive attribute sample (2) is incubated with the purification reagent to perform purification of the next positive attribute sample (2), and the purification procedure for the positive attribute sample (n) is repeatedly performed until the M separated components of all N positive attribute samples are completely purified and collected. According to the above purification procedure, the biomolecules in all of the collected, separated component (m) have a binding affinity range, to the aptamer as a capture ligand, between the stretch forces induced by the applied (m-1)^{th} oscillating magnetic field (fₘ₋₁, Hₘ₋₁) and the m^{th} oscillating magnetic field (fₘ, Hₘ) on the joining pair formed by the aptamer and the biomolecule. This is to apply oscillating magnetic fields to generate stretch forces on the joining pairs formed by aptamers and biomolecules to filter out and collect the biomolecules having a specific range of the binding affinities to the aptamer from the positive attribute samples, thereby, the purification of biomolecules having a specific binding affinity range for the aptamer from the positive attribute sample is achieved. It can be considered as a virtual filter. In addition, the oscillating magnetic field conditions required to detach the biomolecule to be purified from the aptamer during purification, as discussed above, are smaller than the oscillating magnetic field conditions for detaching the aptamer from the biomolecule during aptamer selection, and thus the highest oscillating magnetic field condition (the M^{th} oscillating magnetic field: f_{M} and H_{M}) used in the purification process can be defined by the second oscillating magnetic field (fu and H_{U}) used in the aptamer selection process. Moreover, since all of the biomolecules in the magnetic particle bound complex are detached, the finally collected remaining magnetic particle bound complex does not contain any biomolecules, so it can be provided as a purification reagent for the next positive attribute sample. Furthermore, in order to achieve the function of this virtual filter, the applied oscillating magnetic field must satisfy the following constraints: fₘ₋₁ ≤ fₘ and/or Hₘ₋₁ ≤ Hₘ, and the equality of frequency and strength cannot be applied simultaneously, for example fₘ = fₘ₋₁ and Hₘ = Hₘ₋₁ cannot be applied at the same time. In addition, in order to have the same binding affinity range for the individual separated component (m) purified from all N positive attribute samples using the aptamer as the capture ligand, the oscillating magnetic field conditions of the virtual filters used must be consistent throughout the purification process for all N positive attribute samples.

It is worth noting here that only the mechanical force (stretch force) induced by the oscillating magnetic field is applied during the purification process, and this force is very weak (if the strength of the specific bond is used to estimate, the force is around the level of pN). Without any temperature and chemical influence, the activity of the purified biomolecule is not reduced by the purification process, and the biomolecule purified by this method can retain its activity. This virtual filter can be used for the purification of biomolecules of unknown identity or known identity. The former, including the selection of aptamers as capture ligands for the purification and the purification of biomolecules of unknown identity from the samples using the obtained aptamers, can be carried out in the manner described herein. The latter is relatively simple. Because of knowing the biomolecule identity during aptamer selection, it is only necessary to use this biomolecule as the positive selection sample, and one round of positive selection is needed to select the aptamer which can be used as a detection probe for detection and as a capture ligand for purification using the virtual filter disclosed herein. If a procedure for purifying a specific biomolecule from a sample has been performed once, the range of the oscillating magnetic field (the (m-1)^{th} magnetic field and the m^{th} magnetic field) applied during the purification of the biomolecule is known. Thus, for the subsequent purification using the same aptamer as a capture ligand to purify this particular biomolecule from the sample, it is only necessary to apply this known range of oscillating magnetic fields, without the need to perform an oscillating magnetic field scan, i.e., only one virtual filter is required without using M virtual filters.

It is also worth mentioning that the virtual filter designed according to the range of the binding affinity of the joining pair formed by the aptamer and the biomolecule is based on the mechanism of applying a stretch force to the joining pair, so that any method or facility able to generate the stretch force on the joining pair can be used as a virtual filter to produce the mechanism similar to that of the oscillating magnetic field described above, including but not limited to mechanical forces (e.g., a bound complex consisting of magnetic particles and aptamer-biomolecules in an aqueous solution, via the stretch force induced by the motion driven by the oscillating magnetic field; the fluid dynamic force induced by the rigorous washing in the case where the aptamer-biomolecule complex is attached to a fixed substrate or trapped (e.g., a fluid channel); and a centrifugal force induced by rotation of an aptamer-biomolecule complex attached to a fixed substrate or capture (e.g., a fluid channel) in a disc laboratory (lab-on-a-disc)), electromagnetic force (e.g., a static magnetic force induced by a gradient magnetic field in the case of using magnetic substance and a static electric force induced by an electric field in the case of using charged substance), or any combination of the above forces. In order to perform the mechanism of a virtual filter based on the selecting range of binding affinities, a range of stretch forces generated by varying the oscillating magnetic field, a range of hydrodynamic forces generated by changing the buffer washing speed in the fluid channel, a range of centrifugal forces generated by rotation at different rotational rates of a disc laboratory, or a range of electromagnetic forces produced by varying the strength of the gradient magnetic field and/or electric field, can be used. It should be noted that the selection mechanism of the binding affinity range can be used to select an aptamer having a desired range of binding affinity during aptamer selection and also be used to filter out and collect the biomolecules with binding affinity in a specific range to the aptamer during biomolecule purification.

After completing the purification for all of the chosen positive attribute samples by virtual filters and collecting the M separated components of the N positive attribute samples, the corresponding separated components (the separated components collected under the same magnetic field range) from all of the positive attribute samples are collected for subsequent identification of the identity of the biomolecules contained in the collected separated components. Because the total solution of the collected M separated components contains PBS buffer and purified biomolecules, wherein the total amount of biomolecules is sufficient for the analysis by a mass spectrometer (determined by the number N of positive attribute samples) but the concentration of biomolecules is very low, so the collected separated components are first concentrated. Then, the M concentrated, separated components are separately subjected to polyacrylamide gel electrophoresis to observe the distribution of biomolecule bands on the gel blot. Finally, the desired biomolecule bands are cut out and analyzed by a mass spectrometry in order to sequence the purified biomolecules.

The biomolecules obtained by the purification procedure disclosed by the present invention are the specific disease-related biomolecules and can be used as biomarkers for the disease. In practice, multiple aptamers may specifically bind to the same biomolecule, or an aptamer may specifically bind to multiple biomolecules but have different binding affinities. The former does not have any effect on the results of purification and identification, while the latter, by using a virtual filter for purification to separate biomolecules with different binding affinities to aptamers, will therefore have not any adverse effect on the result of purification and identification. However, a condition that has an adverse impact on the purification results may also occur. An aptamer can bind to multiple biomolecules and have the same binding affinity (e.g., different biomolecules have the same fitting part with the aptamer). At the same time, the size of the different biomolecules is similar (the different types of biomolecules cannot be separated by polyacrylamide gel electrophoresis). In this case, the purification procedure cannot separate these different biomolecules, and different aptamers must be used for the purification. Considering the above situation, it is practical to select multiple aptamers during aptamer selection, and repeat the purification and mass spectrometer analysis disclosed in the present invention for positive attribute samples to confirm possible multiple biomarkers that may be used for the disease diagnosis.

### Example: Purification and Identification of Disease-Related Biomarkers: A Case Study of Non-Small Cell Lung Cancer with Epidermal Growth Factor Receptor Mutation

According to the present invention, the invention relates to a discovery procedure of biomarkers for specific diseases. The invention mainly includes a selection method for selecting aptamers capable of distinguishing the samples of disease patients and non-disease patients from a nucleic acid library and a method for purifying biomolecules with unknown identity in the positive attribute samples by using the aptamers obtained by the selection as capture ligands. These two methods are shown in Figure 1 and Figure 2, respectively. Below, the purification and identification of unknown biomarkers associated with the disease of NSCLC serum samples for EGFR mutation are used as an example to illustrate the specific content of the present invention. For non-small cell lung cancer, the EGFR mutation is located at four exons of the tyrosine kinase domain (exon 18-21), in which is concentrated in exons 19 and 21, accounting for the majority of disease patients, including the deletion mutation of amino acid at codon 746-752 in exon 19 (exon 19 deletion mutation: 45%) and the missense mutation in T-G transversion of amino acid at codon 858 in exon 21 (exon 21 missense mutation: 40%). This example is exemplified by EGFR-mutated NSCLC patients (including EXON 19 and L-858R). The main purpose of which is to explain in detail the techniques disclosed in the present patent application, including the aptamer selection procedure and the biomolecule purification procedure, rather than to present the discovery and definition of disease biomarkers for NSCLC with EGFR mutation. Therefore, only one aptamer obtained by selection is used to demonstrate the purification procedure instead of using multiple aptamers to purify other biomolecules as potential biomarkers of the above disease from the patients' sera. Furthermore, only several biomolecule bands are selected instead of all of the biomolecule bands on the electrophoretic blot, for mass spectrometer analysis to confirm the identity of all of the purified samples. Moreover, the correctness that these biomolecules serve as biomarkers for the above diseases is not further verified by corresponding monoclonal antibodies.

### Experimental Setting of MARAS

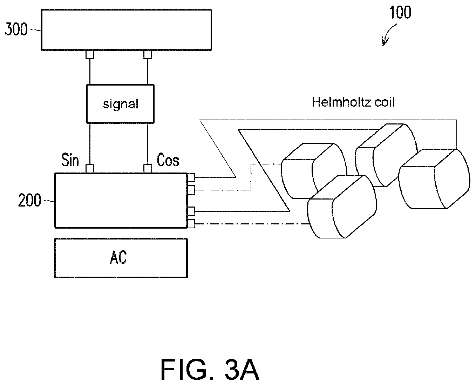

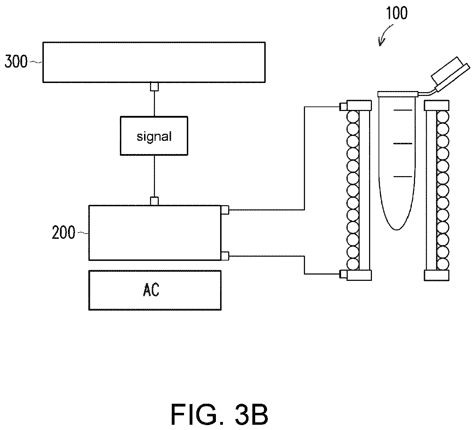

The experimental setting for providing oscillating magnetic fields in the methods of selecting aptamers capable of distinguishing positive and negative samples and purifying biomolecules from the positive attribute samples using the aptamers obtained utilizing MARAS platform is described as below. The experimental setting includes at least two sets of coils **100** for generating an oscillating magnetic field, a power amplifier **200,** and a signal generator **300,** and is operated with a LABVIEW computer program. The oscillating magnetic field used in MARAS may be either a rotating magnetic field as in the case of rotating magnetic field-MARAS (RO-MARAS) or an alternating magnetic field as in the case of alternating magnetic field-MARAS (AC-MARAS). For RO-MARAS, the rotating magnetic field is generated by two sets of Helmholtz coils **100** placed orthogonally. The LABVIEW program **300,** via a NI BNC-2110 capture box, is used to send two signals, cos(ωt) and sin(ωt), into a two-channel power amplifier **200.** These two signals are then amplified equally, which drive two sets of coils simultaneously to produce a rotating magnetic field. The experimental setting is schematically shown in Figure 3A. In the figure, the sample is placed at the intersection of the central lines of two sets of Helmholtz coils **100.** However, other alternative settings may also be used to generate the rotating magnetic field. For AC-MARAS, the magnetic field is generated by a single excitation solenoid **100** driven by a signal generator **300** and current generator unit **200,** as schematically shown in Figure 3B, in which the sample is placed inside the solenoid **100.** It is noted that the setting of Figure 3A can also be used for AC-MARAS if the computer program sends only one signal to one set of Helmholtz coil to generate the AC magnetic field. Furthermore, other alternative settings may also be used to generate the alternating magnetic field. Moreover, other types of oscillating magnetic field may be also applicable, such as an elliptical magnetic field which can be generated by using different amplification factors of the power amplifier **200** for the sine and cosine signals from the signal generator **300** using the setup depicted in Figure 3A. In this example, the rotating magnetic field is applied by the RO-MARAS platform and limited by the specifications of the power amplifier used. In the aptamer selection and biomolecule purification procedures, the strength of all applied rotating magnetic fields is fixed at 14 gauss. Moreover, since the magnetic particles have biological molecules on the surface, they are easily agglomerated with each other under the action of a magnetic field into larger molecular clusters which cause precipitation. Therefore, in the process of applying oscillating magnetic field during the aptamer selection and biomolecule purification, the precipitated clusters are stirred by pipetting every 2.5 minutes for re-dispersion.

### Material preparation

Before performing the aptamer selection procedure using MARAS platform, it is required to prepare the materials. The material preparation includes preparing the random nucleic acid library, and preparing the positive selection samples, negative selection samples, and blind test samples. These preparation steps are summarized in the next section.

### Nucleic acid library and primers

The length of initial nucleic acid library is 60-mer consisting of a randomized 20-mer midsection (N20) and two primers with 20-mer fixed section at both ends. The oligonucleotides of the nucleic acid library chemically synthesized and purified by PolyAcrylamide Gel Electrophoresis (PAGE) are (5'-AGCAGCACAGAGGTCAGATG-N20-CCTATGCGTGCTACCGTGAA-3') (SEQ ID NO: 1). One set of primers, label forward (Lab-F: 5'-AGCAGCACAGAGGTCAGATG-3') (SEQ ID NO: 2) and label reverse (Lab-R: 5'-TTCACGGTAGCACGCATAGG-3') (SEQ ID NO: 3), is used to anneal the 5' and 3' degenerating region of the oligonucleotides during the PCR amplification. 5'-biotin labeled primers, Lab-biotin-F and Lab-biotin-R, with the same sequence as described above, are used to isolate the biotin-forward single strand and forward single strand oligonucleotides from the double strand PCR product, respectively. The universal T7 primer (T7: 5'-TAATACGACTCACTATAGGG-3') (SEQ ID NO: 4) is used to sequence the oligonucleotide of the selected aptamer. All oligonucleotides including library and primers are purchased from MDBio (MDBio, Taiwan). It is mentioned that different lengths and sequences of random nucleic acid library and their corresponding primers can be used without altering the results of this invention.

### Preparation of biotinylated serum-protein conjugated magnetic nanoparticle reagent

The experiment of this example uses five positive selection samples (1=5) of NSCLC patients' sera with EGFR mutation (including EXON 19(2) and L-858R(3)); five negative selection samples (J=5) of NSCLC patients' sera without EGFR mutation; in addition, 18 and 20 NSCLC patients' sera with non-EGFR mutation and EGFR mutation, respectively, which have been confirmed by biopsy pathological analysis, are used as blind test samples (K=38) for blind sample analysis. The clinical data of the serum samples of NSCLC patients used is listed in Tables 1 and 2:

**Table 1**

| Serum clinical data of NSCLC patients of negative and positive selection samples | | | | | | | |
|---|---|---|---|---|---|---|---|
| **Patient** | **Sexuality** | **Age** | **Cell** | **Smoking** | **EGFR** | **TNM** | **Stage** |
| 309(N1) | F | 81 | A | N | Negative | T2aN0M0 | IB |
| 340(N2) | M | 60 | A | Y | Negative | T2aN0M0 | IB |
| 372(N3) | M | 66 | A | Y | Negative | T1bN1M0 | IIA |
| 416(N4) | M | 67 | A | Y | Negative | T1bN0M0 | IA |
| 452(N5) | F | 74 | A | N | Negative | T1aN0M0 | IA |
| 234(P1) | F | 62 | A | N | L-858R | T1bN2M0 | IIIA |
| 322(P2) | F | 63 | A | N | L-858R | T4N3M0 | IIIB |
| 365(P3) | F | 60 | A | N | L-858R | T1aN0M0 | IA |
| 98(P4) | F | 59 | A | N | Exon-19 | T2aN1M0 | IIA |
| 272(P5) | F | 79 | A | N | Exon-19 | T2aN0M0 | IB |

**Table 2**

| Serum clinical data of NSCLC patients of blind test samples | | | | | | | |
|---|---|---|---|---|---|---|---|
| **Patient** | **Sexuality** | **Age** | **Cell** | **Smoking** | **EGFR** | **TNM** | **Stage** |
| 1 | M | 80 | A | Y | Negative | T4N3M1b | IV |
| 2 | F | 77 | A | N | L-858R | T2aN0M1b | IV |
| 3 | M | 75 | A | Y | Negative | T1bN3M1b | IV |
| 4 | F | 74 | A | N | Negative | T4N3M1a | IV |
| 9 | F | 47 | A | N | Negative | T2aN2M0 | IV |
| 12 | F | 45 | A | N | Negative | T1bN2M1a | IV |
| 13 | F | 71 | A | N | L-858R | T21N0M1a | IV |
| 17 | F | 61 | A | N | L-858R | T2aN0M0 | IIIB |
| 21 | F | 78 | A | N | L-858R | T1bN0M1b | IV |
| 22 | F | 84 | A | N | L-858R | T2aN0M0 | IB |
| 29 | F | 44 | A | N | Negative | T3N1M1b | IV |
| 31 | F | 75 | A | N | L-858R | T1bN0M0 | IA |
| 35 | F | 77 | A | Y | Negative | T2aN0M0 | IB |
| 42 | M | 61 | A | Y | L-858R | T2aN3M0 | IIIB |
| 73 | M | 58 | A | Y | Negative | T2bN0M0 | IIA |
| 75 | F | 75 | A | N | L-858R | T2aN3M0 | IIIB |
| 78 | F | 64 | A | N | L-858R | T2aN1M0 | IIA |
| 99 | F | 56 | A | N | Negative | T2aN0M0 | IB |
| 102 | F | 74 | A | N | L-858R | T2aN0M0 | IB |
| 116 | F | 58 | A | N | Negative | T1aN0M0 | IA |
| 141 | M | 88 | A | N | Negative | T2bN0M0 | IIA |
| 154 | F | 42 | A | N | Negative | T1bN0M0 | IA |
| 176 | M | 45 | A | N | L-858R | T2aN2M0 | IIIA |
| 177 | F | 51 | A | N | L-858R | T2aN2M0 | IIIA |
| 178 | M | 52 | A | N | L-858R | T2aN1M0 | IIA |
| 271 | F | 82 | A | N | Negative | T2aN0M0 | IB |
| 288 | F | 91 | A | N | Negative | T1bN0M0 | IA |
| 307 | M | 81 | A | Y | L-858R | T2aN2M0 | IIIA |
| 318 | M | 62 | A | Y | Negative | T2aN1M0 | IIA |
| 324 | F | 49 | A | N | L-858R & T-790M | T1aN0M0 | IA |
| 326 | M | 59 | A | Y | Negative | T1aN0M0 | IA |
| 329 | F | 70 | A | Y | Negative | T4N1Ma | IV |
| 335 | F | 62 | A | Y | Negative | T3N2M0 | IIIA |
| 358 | F | 77 | AS | N | L-858R | T2aN3M0 | IIIB |
| 390 | M | 75 | A | Y | L-858R | T2aN3M1a | IV |
| 396 | F | 65 | A | N | L-858R | T2aN0M0 | IV |
| 399 | F | 78 | A | N | L-858R | T1aN0M0 | IV |
| 460 | M | 50 | A | Y | L-858R | T2N2M1b | IV |

The amount of protein in the individual clinical serum samples in Tables 1 and 2 above is individually determined using a Bio-Rad protein assay (Bio-Rad, Taiwan) with an enzyme immunoassay analyzer (ELISA Reader, Molecular Devices, Taiwan) at an absorbance value of 570 nm. The total protein amount of each sample is controlled at 100 µg and a biotinylation kit (EZ-LinkTM Sulfo-NHS-Biotinylation kit, Thermo Scientific; Nanosep® Centrifugal Devices, Life Science, USA) is used for biotinylation. Biotin is labeled on the proteins of individual clinical serum samples according to the manufacturer's instructions, followed by incubating 50 µL streptavidin-coated, bio-functionalized magnetic nanoparticle reagents (SA-MNP reagent) and biotinylated serum samples at 4°C reacted for more than 16 hours to prepare biotinylated serum protein-magnetic nanoparticle reagent (sample reagent), including 5 positive selection sample, 5 negative selection sample and 38 blind test sample reagents (PS-MNP, NS-MNP, and BS-MNP reagents) which are stored in a refrigerator at 4°C for the future experiments. SA-MNPs are dispersed in PBS buffer to form SA-MNP reagent and purchased from Magqu Biotechnology (Magqu, Taiwan). The mean hydrodynamic diameter of the SA-MNPs in the reagent is 50 nm and a concentration of SA-MNP is 0.3 emu/g. Before using SA-MNPs and sample-MNPs (including PS-MNPs, NS-MNPs, and BS-MNPs), these reagents should be magnetically separated and washed at least twice with PBS buffer for the subsequent experiments.

### Procedure for selecting aptamers with the ability to distinguish the disease patients by RO-MARAS

A 1 nM single strand nucleic acid library is dissolved in 100 µL of PBS buffer, then heated, and rapidly cooled as described above for the formation of specific secondary or tertiary structures. After completion, the five types of negative selection sample reagents prepared in advance are washed twice with PBS buffer and magnetically separated to obtain NS-MNPs (negative selection samples), separately. The NS-MNPs₍₁₎ and the single strand nucleic acid library are placed in a rotary mixer to well mix for 1 hour. A magnetic separation with a magnetic stand is performed, the supernatant is retained, and the oligonucleotides that bind to any of the proteins in NS-MNPs₍₁₎ are removed. Then the collected supernatant containing the remaining oligonucleotides is incubated with the next negative selection sample, NS-MNPs₍₂₎, and the above steps are repeated J (5) times to complete the negative selection for the remaining NS-MNPs₍₂₎ to NS-MNPs₍₅₎, sequentially. The final supernatant is purified by using a DNA Extraction Miniprep System (Gel/PCR DNA Isolation System, Viogene, Taiwan) and re-dispersed in 100 µL of PBS buffer to complete the negative selection.

Next, the positive selection is performed. The re-dispersed supernatant containing the remaining oligonucleotides after negative selection and purification is heated and rapidly cooled as described above for the formation of the oligonucleotides into specific secondary or tertiary structures, and the five types of positive selection sample reagents prepared above are washed twice with PBS buffer and magnetically separated to obtain PS-MNPs (positive selection samples), separately. The supernatant of the remaining nucleic acid library after negative selection is then incubated with the positive selection sample (PS-MNPs₍₁₎) of the EGFR-mutated NSCLC patient, and placed on a rotary mixer for 1 hour, followed by a magnetic separation. After removing the supernatant, the PS-MNPs₍₁₎ and oligonucleotide bound mixture is collected, washed several times, and dissolved with PBS buffer (100 µL). Then the bound mixture solution is subjected to a specific rotating magnetic field condition (magnetic field frequency: 27 KHz, magnetic field strength: 14 gauss) for MARAS selection, to remove oligonucleotides with low binding affinity to PS-MNPs(i). Since there are biomolecules on the surface, magnetic nanoparticles are easy to agglomerate with each other and form large clusters resulting in precipitation, therefore, the magnetic particle bound mixture is stirred by pipetting every 2.5 minutes. Magnetic separation is then performed to remove the supernatant. The PS-MNPs₍₁₎ and the oligonucleotide bound mixture is collected and washed several times with PBS buffer. The remaining oligonucleotides on the PS-MNPs₍₁₎ and oligonucleotide bound mixture are the desired oligonucleotides having a strong binding affinity to the proteins bound on PS-MNPs₍₁₎. The bound mixture is dispersed with 20 µL of ddH₂O, and then heated to 95°C for 10 minutes to detach the oligonucleotides. A magnetic separation is performed to collected supernatant containing the detached oligonucleotides which are then purified with a DNA Extraction Miniprep System, re-dispersed with 100 µL of PBS buffer, heated, and rapidly cooled as described above for the formation of the oligonucleotides into specific secondary or tertiary structures. After the formation of the structures, the oligonucleotides are incubated with PS-MNPs₍₂₎, and the above positive selection steps are repeated I (5) times to complete the positive selection for the remaining PS-MNPs₍₂₎ to PS-MNPs(5), sequentially. The finally obtained oligonucleotides are the aptamers which do not bind to all proteins on NS-MNPs₍ⱼ₎ (j = 1 to 5) and only bind to proteins commonly contained in PS-MNPs₍ᵢ₎ (i = 1 to 5) with high binding affinity. The experimental procedure is shown in Figure 1. The loop on the right in the figure is the loop of the negative selection. At this time, it is not necessary to use the MARAS, but only to exclude the single strand oligonucleotides that bind to the NS-MNPs of all five types of negative selection samples. Next, there is a positive selection loop on the left. At this time, a competitive mechanism provided by MARAS is used to allow the single strand oligonucleotides with strong binding affinity to remain on the PS-MNPs. These single strand oligonucleotides (aptamers) are then eluted from the MNPs by heating and purified for subsequent experiments. While the use of five types of negative selection samples and five types of positive selection samples is mainly to improve the specificity and sensitivity of the selected aptamers to reduce the problems of false negatives and false positives during the subsequent detection and purification.

### Sequencing oligonucleotide aptamer sequences

The selected aptamers are amplified by PCR using Lab-F and Lab-R primers and the PCR reaction is carried out to contain 1.25 units of DNA polymerase (Invitrogen Life Technologies, Grand Island, NY, USA), 0.1 mM dNTPs, 0.5 mM MgSO₄, and 0.5 nM primers performed under the following conditions: 10 minutes at 94°C; 40 seconds at 94°C, 40 seconds at 57°C, 40 seconds at 72°C, 35 cycles; 10 minutes at 72°C. The PCR product is purified by using a DNA Extraction Miniprep System. The purified product is subcloned into pGEM-T Easy vector (Promega, Madison, WI, USA). The cloning procedure is performed according to the manufacturer's instructions. The plasmids of three randomly-selected colonies are purified by using a High-Speed Plasmid Mini Kit (Geneaid, Taipei, Taiwan). The plasmid is sequenced by using an Applied Biosystems PRISM 3730 DNA automatic sequencer and a Big Dye terminator cycle sequencing kit (Foster City, CA, USA). The aptamers selected by the MARAS method bind to disease-related biomolecules of EGFR-mutated NSCLC. After PCR amplification and colonization, the sequencing results are listed in Table 3.

**Table 3**

| Sequence of the N20 region of the aptamer selected by MARAS | |
|---|---|
| aptamer clone name | N20 sequence |
| Aptamer37 | GGCCCTCCAGCCATGCTGTG (SEQ ID NO: 5) |
| Aptamer44 | GGTGGCAGCGTAAGGGCAAT (SEQ ID NO: 6) |
| Aptamer48 | GTCTCGGCCCACCCTCGCGA (SEQ ID NO: 7) |

### Preparation of the selected forward single strand aptamers

In order to confirm whether the selected aptamers can actually bind to the selection control group (positive selection samples and negative selection samples), it is necessary to reverse validate the selected aptamers with the PS-MNPs and NS-MNPs. The oligonucleotide plasmids of the three aptamers listed in Table 3 are used as templates for PCR amplification using Lab-F/Lab-biotin-R as primers (the reaction conditions are the same as described above), and the amplified product is purified by using a DNA Extraction Miniprep System and re-dispersed in 100 µL of PBS buffer, individually. Then, the PCR product is incubated with SA-MNPs for 2 hours, the supernatant not bound to SA-MNPs is removed by magnetic separation, and the magnetic nanoparticle bound complex is dispersed in 100 µL of PBS buffer. A freshly prepared 0.15 M NaOH is added for 4 minutes to destroy the double strand bond of the oligonucleotides. After magnetic separation, the supernatant is collected, and the magnetic particles bound with biotinylated reverse single strand oligonucleotides are removed. The collected supernatant is added to 100 mL of 100% alcohol, placed in a -80°C freezer for 2 hours, then centrifuged at 12,000 rpm for 15 minutes, removed the supernatant, and added 1 mL of 75% of alcohol. The salt is removed by centrifugation at the same speed for 15 minutes. After removing the supernatant, it is placed in a dry bath at 70°C to volatilize residual water and alcohol. After drying, 100 µL of PBS buffer is added to dissolve the precipitate to obtain the purified forward single strand aptamers. After purification of the single strand aptamers, a spectrophotometer (NanoDrop 2000c, Thermo Fisher Scientific, Wilmington, DE, USA) is used to determine the concentration, and the three aptamers are individually diluted to a fixed concentration of 10 nM in PBS buffer for use in subsequent reverse validation and blind sample analysis.

### Reverse validation of the binding of the selected aptamers and the selection control group by q-PCR

100 µL of three aptamers (Aptamer37, Aptamer44, and Aptamer48) diluted to a concentration of 10 nM is heated and rapidly cooled as described above to form specific secondary or tertiary structures, and separately incubated with the selection control groups, five types of PS-MNPs₍ᵢ₎ (i =1∼5) and five types of NS-MNPs₍ⱼ₎ (j = 1∼5), at room temperature for 60 minutes. A magnetic separation is performed to remove the supernatant, and then the magnetic particle bound complex is collected and washed several times with PBS buffer to completely remove the unbound aptamers. Finally, the magnetic particle bound complex is dispersed with 10 µL of ddH₂O and heated to 95°C for 10 minutes to break the bond between the aptamer and the magnetic particles, and the supernatant containing the aptamers is collected by magnetic separation.

The quantity of the aptamers in the collected supernatant containing the aptamers is then measured by q-PCR (StepOne™ Real-time PCR system, Applied Biosystems) in duplicate, and the fluorescent strength emitted by each cycle is analyzed. When the released strength reaches the system preset threshold (Threshold), then the number of PCR cycles is set to be Ct (Threshold cycle), so the resulting Ct value is inversely proportional to the quantity of the starting aptamers in the supernatant. Each q-PCR reaction mixture is 10 µL containing 5 µL of SYBR Green PCR master mix (Applied Biosystems), 0.5 µL of Lab-F/Lab-R (0.5 nM), and 4 µL of the aforementioned, collected supernatant. The reaction conditions are as follows: 5 minutes at 95°C; 40 seconds at 94°C, 40 seconds at 60°C, and 40 seconds at 72°C, 40 cycles; 10 minutes at 94°C. The formula for estimating the quantity of the aptamer by the Ct value is: the relative expression level of the oligonucleotide = 2^{-Ct}.

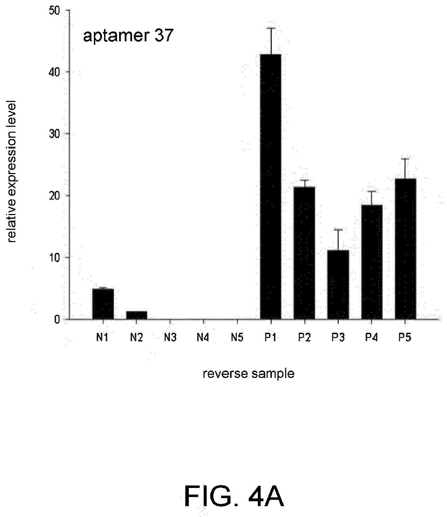

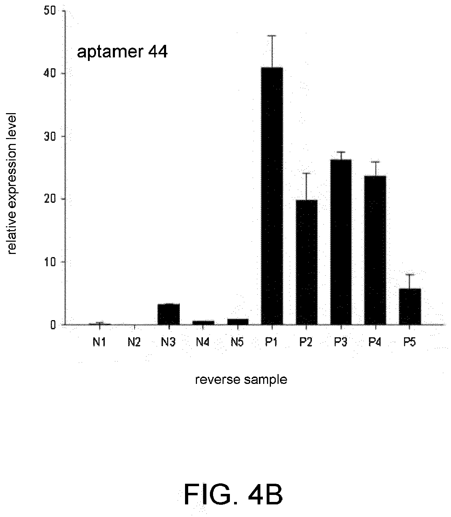

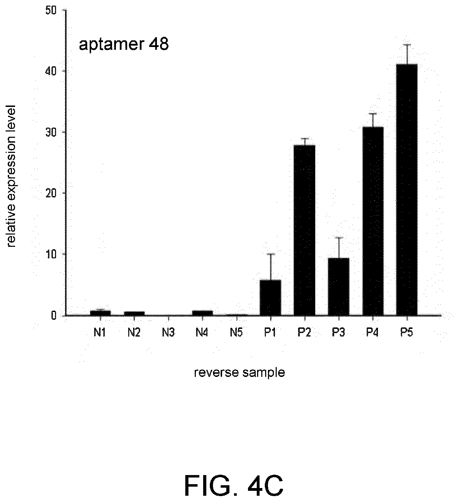

To confirm whether the selected aptamers are indeed capable of identifying the serum of NSCLC patients with and without EGFR mutation, a reverse validation is performed to confirm the selection procedure. The results are as shown in Figures 4A-4C. These figures show that the amount of binding of the three selected aptamers to the selection control groups corresponds to the relative level of expression, and there is a significant difference between the selection samples of NSCLC patients with and without EGFR mutation. Thus, the correctness of the execution of the aptamer selection method disclosed in the flow chart of Figure 1 is confirmed.

### Blind sample analysis of blind test samples by q-PCR method for the obtained aptamers

Further, the three aptamers are analyzed by blind sample analysis to detect whether there is any unknown biomolecules in which the aptamer can bind in the blind test samples, and similarly it is carried out by the q-PCR method (the reaction conditions are the same as described above) in duplex. First, a 100 µL fixed concentration (10 nM) of the aptamer in PBS buffer is heated and then rapidly cooled as described above to form a specific secondary or tertiary structures, and then incubated individually with each of BS-MNPs₍ₖ₎ (k= 1-38). After uniformly mixing, the mixed solution is kept at room temperature for 60 minutes, the supernatant is removed by magnetic separation, and then the BS-MNP bound complex are washed several times and dispersed in 100 µL of PBS buffer. The BS-MNP bound complex solution is subjected to a specific rotating magnetic field (magnetic field frequency: 27 KHz, magnetic field strength: 14 gauss) for 10 minutes in order to detach the portion of aptamers with nonspecific and low binding affinity bound to BS-MNPs₍ₖ₎ from BS-MNP₍ₖ₎ bound complex. The supernatant containing the detached aptamers is removed and the BS-MNP₍ₖ₎ bound complex is retained by magnetic separation. Finally, the BS-MNP₍ₖ₎ bound complex is re-dispersed in 10 µL of ddH₂O, and heated to 95°C for 10 minutes to break the bond between the aptamers and the magnetic particles. A magnetic separation is performed to remove the magnetic particles and collect the supernatant. The amount of aptamers bound to biomolecules of each individual BS-MNPs₍ₖ₎ in the collected supernatants is then analyzed by q-PCR in duplex as described above and expressed as the relative expression level.

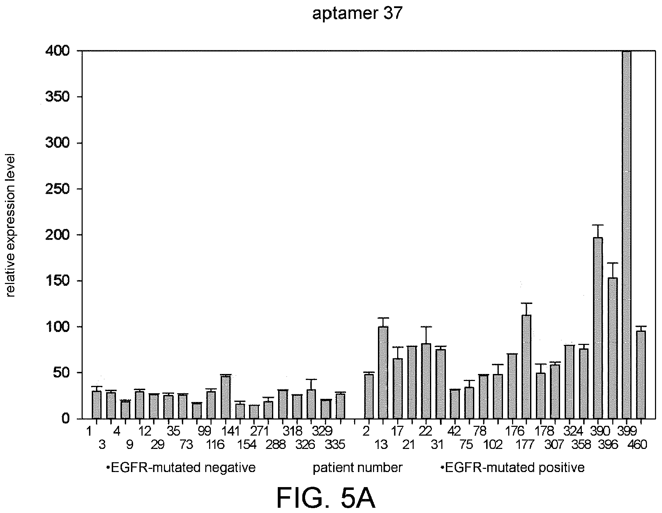

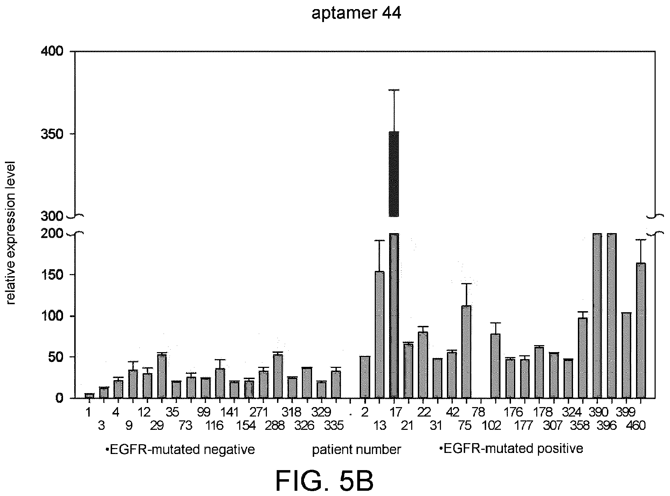

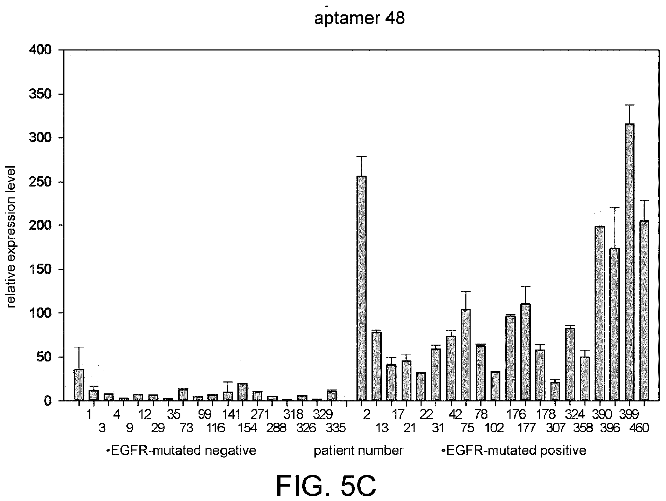

The aptamers (Aptamer37, Aptamer44, and Aptamer48) that are selected and validated by reverse validation are further used for blind sample analysis of blind test samples, that is, the BS-MNPs prepared using the sera of NSCLC patients other than the selection control group are analyzed blindly, and the results are expressed by the relative expression level of q-PCR analysis. The q-PCR results are compared with the results of the biopsy pathological analysis of NSCLC patients' sera in the blind test group. The results are shown in Figures 5A to 5C. From the result, it is found that each aptamer has different degrees of binding to different blind test samples, indicating that the binding amount of each aptamer to unknown biomolecules in a serum sample of a different patient is different. It can also be found that the relative expression of the positive blind test samples is generally higher than that of the negative blind test samples, and the positive blind test sample with high relative expression of each individual aptamer is not identical. The former shows that the selected aptamers have the ability to distinguish NSCLC patients with and without EGFR mutation, while the latter shows that the biomolecules in the serum of positive patients bound by individual aptamer may vary. Furthermore, all aptamers in the negative blind test samples also have lower and different degrees of relative expression relative to the positive blind test samples, which should be due to the presence of biomolecules bound by the aptamer in the negative and positive blind test samples. However, the concentration of these biomolecules in the serum of the positive patients is much higher than that in the serum of the negative patients. It is worth mentioning that because of the run out of the serum of some blind test samples, the relative expression by q-PCR of some aptamer bound to the blind test sample has no data. Also, the relative expression of the aptamers for a certain positive blind test sample is low, it is probably because the positive blind test sample is collected for the patient after medical treatment that cannot be confirmed because there is no complete medical record. However, these two situations do not affect the interpretation of the results. Further the relative expression of individual aptamers associated with BS-MNPs in blind sample analysis is compared with the factors of the patients' clinical data, including age, sex, cancer stage, presence or absence of smoking, and EGFR mutation, using Mann-Whitney U Test for statistical analysis. The comparison result is given in Table 4. It can be clearly seen from the table that the selected aptamers are significantly associated with the presence or absence of EGFR mutation (p-value <0.05) and insignificantly correlated with other factors in the clinical data. The results of the statistical analysis further verify the ability of the selected aptamers to distinguish between samples of NSCLC patients with and without EGFR mutation.

**Table 4**

| Correlation between the binding amount of aptamer and clinical factors of blind test samples | | | | | | | |
|---|---|---|---|---|---|---|---|
| | | Apatamer37 | | Aptamer44 | | Aptamer48 | |
| | No. of case | mean±SD | *p*-value | mean±SD | *p*-value | mean±SD | *p*-value |
| Age | | | | | | | |
| <65 | 18 | 47.60 ± 29.87 | 0.206 | 62.66 ± 79.13 | 0.317 | 14.52 ± 15.54 | 0.965 |
| ≥65 | 20 | 81.98 ± 96.46 | | 73.22 ± 62.44 | | 28.44 ± 48.74 | |
| Gender | | | | | | | |
| Male | 12 | 56.51 ± 49.85 | 0.816 | 57.09 ± 52.51 | 0.545 | 19.74 ± 23.50 | 0.914 |
| Female | 26 | 69.94 ± 83.51 | | 73.36 ± 77.22 | | 22.82 ± 42.39 | |
| Stage | | | | | | | |
| I/II/IIIA | 20 | 48.68 ± 33.64 | 0.243 | 54.55 ± 57.02 | 0.308 | 11.44 ± 12.04 | 0.152 |
| IIIB/IV | 18 | 79.47 ± 93.84 | | 79.29 ± 78.67 | | 30.26 ± 47.66 | |
| Smoking Status | | | | | | | |
| Negative | 26 | 73.31 ± 82.29 | 0.081 | 76.22 ± 75.77 | 0.061 | 23.99 ± 42.01 | 0.185 |
| Positive | 12 | 49.20 ± 51.34 | | 50.89 ± 54.57 | | 17.18 ± 24.41 | |
| EGFR mutation | | | | | | | |
| Negative | 18 | 27.66 ± 7.46 | <0.001 | 31.15 ± 13.83 | <0.001 | 7.73 ± 5.00 | 0.002 |
| Positive | 20 | 99.93 ± 89.66 | | 101.59 ± 83.13 | | 34.55 ± 48.03 | |

### Purification of unknown biomarkers in positive attribute samples

In this example, the Aptamer37 aptamer is used as a capture ligand to demonstrate the purification of biomolecules that may be served as biomarkers for EGFR mutation in NSCLC, from positive attribute blind test samples. The positive attribute samples (patient number: Nos. 22, 31, 177, and 396) which have high relative expression to Aptamer37 are chosen for purification. Purification reagent must be prepared prior to purification. The reagent is prepared as follows.

### Preparation of purification reagent using the selected aptamer (Aptamer37) as a capture ligand

The aptamer (Aptamer37) is used as a template, and PCR reaction is performed using Lab-biotin-F/Lab-R. The PCR reaction conditions are as described above. After the reaction, the PCR product is purified by using a DNA Extraction Miniprep System and re-dispersed with 100 µL of PBS buffer, followed by addition of SA-MNPs for 2 hours. Since the PCR product is double strand aptamers hybridized by forward and reverse strands of which the 5-termini of the forward single strand is biotinylated, so the double strand aptamers bind to SA-MNPs. A magnetic separation is performed to remove the supernatant containing the substance not bound to SA-MNPs, and retain the magnetic particle bound complex. The bound complex is then dispersed in 100 µL of PBS buffer and a freshly prepared 0.15 M NaOH is added for 4 minutes to destroy the double strand bond of the aptamers. After removing the supernatant (containing reverse single strand aptamers) by magnetic separation, the magnetic nanoparticles and the forward single strand aptamer bound complex is re-dispersed using 100 µL PBS buffer to complete the preparation of the aptamer purification reagent which is then stored in a refrigerator at 4°C for further use.

### Purification of disease-related unknown biomarkers in blind test samples with positive attribute

In the present purification example, the aptamer purification reagent prepared above is used to purify proteins of unknown identity associated with a disease having NSCLC with EGFR mutation from positive attribute blind test samples of disease patients, 22, 31, 177, and 396. In this experiment, the above-mentioned aptamer purification reagent is used to purify unknown biomarkers in sera in a fashion with a small quantity per time and multiple rounds. A 100 µL of the serum of the positive attribute blind test sample (positive attribute sample (1)) is mixed with the previously prepared aptamer purification reagent at room temperature for one hour, then a magnetic separation is performed to remove the supernatant and collect the aptamer magnetic nanoparticle bound complex. The bound complex is washed several times with PBS buffer to wash away the other substances that are not bound but remaining in the tube wall, and then dispersed in 100 µL of PBS buffer. The bound complex solution is subjected to an applied initial rotating magnetic field with a fixed magnetic field strength of 14 gauss (Ho) and a magnetic field frequency of 3 KHz (f₀) for 10 minutes, and the precipitated magnetic particle clusters are re-dispersed by pipetting every 2.5 minutes as described above. A magnetic separation is performed to collect the supernatant containing the fraction detached from the aptamer magnetic nanoparticle bound complex and the remaining aptamer magnetic nanoparticle bound complex is retained for the purification procedure by applying the rotating magnetic field. As shown in Figure 2, the initial rotating magnetic field (f₀, H₀) is mainly used to remove biomolecules that bind to the aptamer nonspecifically or with low binding affinity, and in this embodiment, the supernatant of this fraction is also retained which is to be presented on subsequent electrophoresis to demonstrate that this step can exclude a large number of interfering biomolecules in the positive attribute sample for subsequent purification and mass spectrometer analysis. The retained aptamer magnetic nanoparticle bound complex is dispersed in 100 µL PBS buffer, followed by the next round of applying a rotating magnetic field with a magnetic field strength of 14 gauss (H₁) and a magnetic field frequency of 6 KHz (f₁) for 10 minutes. The precipitated magnetic particle clusters are re-dispersed by a pipetting every 2.5 minutes as described above. The supernatant containing the fraction (separated component (1)) detached from the aptamer magnetic nanoparticle bound complex is collected by magnetic separation, and the remaining aptamer magnetic nanoparticle bound complex is retained and dispersed in 100 µL PBS buffer as the sample of the positive attribute sample (1) for the next round of the purification procedure by applying the next rotating magnetic field (f₂, H₂). The same purification procedure is repeated (magnetic field conditions: 6 K, 9 K, 12 K, 15 K, 18 K, 21 K, 24 K, 27 KHz; 14 gauss) and each fraction (m, m = 1 to M (= 8)) is collected. After the last round (M) of purification for the same positive attribute sample (1), the retained aptamer magnetic nanoparticle bound complex is dispersed in 100 µL PBS buffer and can continuously be used as an aptamer purification reagent to repeatedly perform the purification procedure on the next positive attribute sample (2) until the completion of the purification procedure for all positive attribute samples (n, n = 1 to N). After completing the purification procedure for all positive attribute samples (n), each set of corresponding separated component (m) collected from the purification procedure of each positive attribute sample (n) is pooled, and finally a MicrosepTM Advance Centrifugal Devices (MCP003C41 3K, PALL, Taiwan) is used separately to concentrate the separated component (m) of each group for subsequent gel electrophoresis of polyacrylamide. The purification process is shown in Figure 2.

### Polyacrylamide gel electrophoresis of unknown proteins

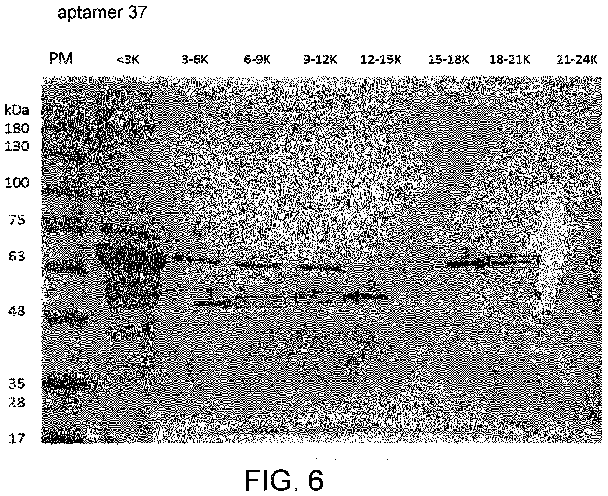

This electrophoresis is carried out using a gel casting and an electrophoresis apparatus of a CAVOY MP-8000 Mini P-4 vertical electrophoresis system (Scientific Biotech Corp., Taiwan). After the device is assembled, a specific concentration of a separating gel solution is added, and an appropriate amount of alcohol is added to flatten the gel surface. After the separating gel is solidified, the upper layer of alcohol is poured off, dried by a filter paper, and then the stacking gel solution is added. Then a suitable size comb (comb) is inserted, and placed in an electrophoresis tank after the gel is solidified, and the running buffer (10x running buffer) is injected. The separated component (m) after concentration and the protein marker of standard molecular weight are separately mixed with 5x sample buffer in a ratio of 3:1, then heated at 98°C for 10 minutes, respectively, and injected into the cogging of the gel. The electrophoresis is started with a fixed voltage of 80 volts. When the protein samples move to the separating gel, the voltage is raised to 100 volts for electrophoresis. When the blue dye moves to the bottom of the gel, the electrophoresis is finished. The gel film is taken out, washed 3 times with deionized water, and then stained with protein dye (instant blue, GeneMark, Taiwan) for 1 hour followed by the removal of the instant blue solution. The gel film is de-dyed with deionized water. After the water does not change color, the distribution of the protein bands on the gel film can be observed, and finally the band of the desired protein size is cut out for sequencing. All of the gel and buffer solutions used are shown in Table 5. After the supernatants collected under each frequency range (protein or biomarker purified under the frequency range) are concentrated, the electrophoresis results of proteins purified using virtual filters simulated by applying magnetic field frequency ranges can be obtained by a polyacrylamide gel electrophoresis, as shown in Figure 6. Figure 6 shows the purification result by the frequency range scan using Aptamer37. In Figure 6, the first column is the protein marker (PM), the second column is the large number of interfering biomolecules to be excluded (< 3 KHz; 14 gauss), and the third to the ninth column are the separated component (1) (3-6 KHz; 14 gauss) to the separated component (7) (21-24 KHz; 14 gauss). The last separated component (8) is not juxtaposed because there is no biomolecule. The second column shows that a large number of interfering biomolecules from the positive attribute samples can be excluded by applying the initial magnetic field (magnetic field conditions: f₀ = 3 KHz, H₀ = 14 gauss), while the other columns (columns 3 to 9) appear that the use of the magnetic field frequency range as a virtual filter can purify possible biomarkers related to disease with EGFR-mutated NSCLC from positive attribute samples.

**Table 5**

| Polyacrylamide gel and buffer for electrophoresis | |
|---|---|
| **Acrylamide Gels for SDS-PAGE** | |
| **6% Stacking gel** (**5** mL) | |
| H₂O | 2.6 mL |
| Acrylamide/Bis-acrylamide (30%/0.8% w/v) | 1 mL |
| 0.5 M Tris-HCl, pH 6.8 | 1.25 mL |
| 10% (w/v) ammonium persulfate (APS) | 50 µL |
| 10% (w/v) SDS | 50 µL |
| TEMED | 5 µL |

| **12% Separating gel** (**10** mL) | |
|---|---|
| H₂O | 3.3 mL |
| Acrylamide/Bis-acrylamide (30%/0.8% w/v) | 4.0 mL |
| 1.5M Tris (pH=8.8) | 2.5 mL |
| 10% (w/v) ammonium persulfate (APS) | 100 µL |
| 10% (w/v)SDS | 100 µL |
| TEMED | 10 µL |

| **10X Running buffer (500 mL)** | |
|---|---|
| Tris-HCl | 15.0 g |
| Glycine | 72.0 g |
| SDS | 5.0 g |

| **5X Sample buffer** | |
|---|---|
| SDS | 10% w/v |
| beta-mercapto-ethanol | 10 mM |
| Glycerol | 20 % v/v |
| Tris-HCl, pH 6.8 | 0.2 M |
| Bromophenolblue | 0.05% w/v |
| SDS | 10% w/v |

### Sequencing of unknown proteins in gel electrophoresis by mass spectrometer

The specific single protein bands purified by the aptamer purification reagent are performed by Biochem® Biotechnology® (AllBio Science Inc., Taiwan) for mass spectrometry analysis using Bruker autoflex® III MALDI-TOF mass spectrometer (Bruker, Taiwan). In this case, only three protein bands are exemplarily selected for mass spectrometry.

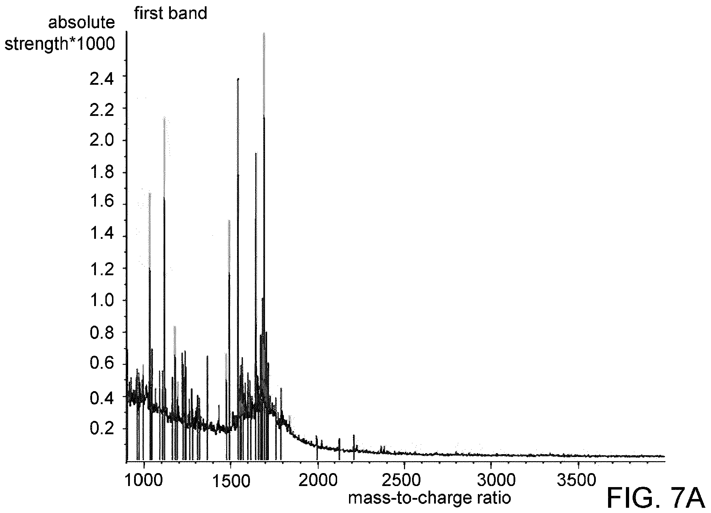

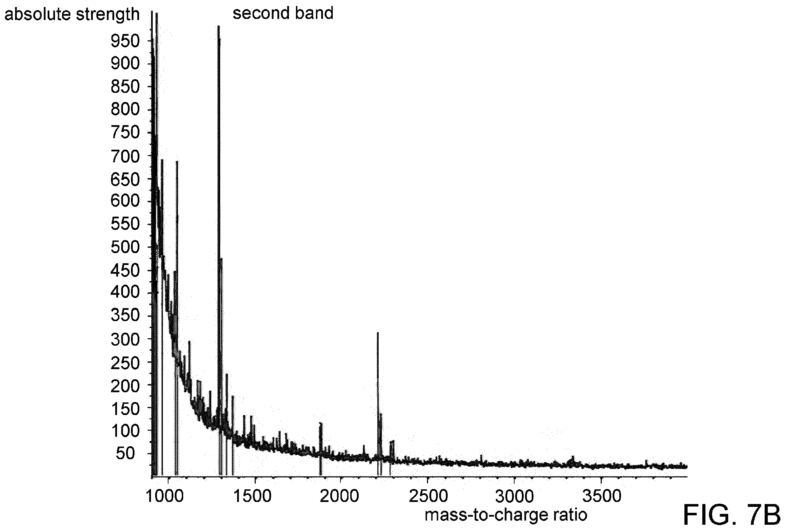

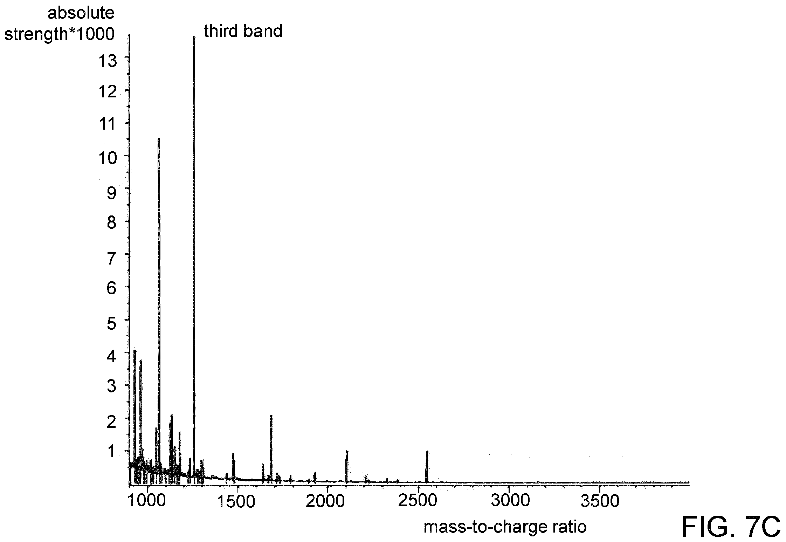

The spectra analyzed by the mass spectrometer of the three purified protein bands indicated by the arrows in Figure 6 above are shown in Figures 7A-7C, respectively. The results are compared with the protein database. The compared result of the first band is to be the hypothetical protein 1308_05599 (52 KDa) with a score of 82, the second band is to be the hypothetical protein G7K_6558-t1 (58 KDa) with a score of 78, and the third band is to be ABC transporter (72 KDa) with a score of 73.

In summary, aptamers capable of distinguishing the samples of NSCLC disease patients with and without EGFR mutation can be selected from a nucleic acid library by using the MARAS platform, and the purification reagents using aptamers as capture ligands, obtained by the selection procedure, are further prepared. The biomolecules associated with the disease can be purified from positive attribute samples by using the purification reagents, of which virtual filters, capable of filtering biomolecules with fixed ranges of binding affinities to the aptamer, simulated by applying magnetic field ranges are used. Then a mass spectrometry can be used to sequence and confirm the identities of the purified biomolecules. If further validated by the corresponding monoclonal antibody, biomolecules may be discovered as biomarkers for NSCLC disease with EGFR mutation. The biomarker discovery procedure disclosed in the present invention includes two main parts in addition to the well-known biochemical related technologies (such as PCR, q-PCR, electrophoresis, and mass spectrometry analysis): (1) the selection of aptamers capable of distinguishing samples of disease patients from nucleic acid libraries using the MARAS platform, and (2) the purification of biomolecules having certain binding affinity range to the aptamers (the obtained aptamers) as capture ligands from positive attribute samples using virtual filters simulated by applying magnetic field ranges. In the former, because the composition of the sample of individual patients may be different, samples of multiple non-disease patients are used as negative selection samples to remove the oligonucleotides bound to non-disease-related components of the samples of non-disease patients from the nucleic acid library in order to reduce the effect of false positives during detection and purification. Furthermore, multiple positive patient samples are used for positive selection, so that the aptamers obtained by selection can only conjugate with the biomolecules commonly existed in all positive patient samples in order to reduce the effects of false negatives during detection and purification, thereby enabling the obtained aptamers to have the ability to distinguish between disease and non-disease patients. And in the latter, the virtual filter used provides the stretch force on the joining pair formed by the capture ligand (the aptamer obtained by selection) and the biomolecules by an applied oscillating magnetic field, so when applying an upper and lower cutoff oscillating magnetic fields, the range of binding affinities of the purified biomolecules to the capture ligands are defined by the upper and lower cutoff oscillating magnetic fields. The range of binding affinities of the purified biomolecules to the capture ligands will be different if the applied oscillating magnetic field range is different. That is, the different biomolecules are purified. The samples of multiple positive attribute patients are purified by virtual filters to obtain sufficient amounts of individual biomolecules, and the individual separated components from the purification are concentrated for mass spectrometry sequencing. In addition, the purification technique of the virtual filters simulated by applying oscillating magnetic field ranges disclosed in this patent specification can also be used to purify unknown or known biomolecules (targets) from positive attribute samples, and the activity of the purified biomolecules is not reduced by the execution of the purification procedure.

## Claims

1. A method of selecting aptamers, wherein each selected aptamer is capable of distinguishing positive samples and negative samples, comprising:
a) providing a library of oligonucleotides with random nucleotide sequences denoted a random sequence library, and heating and quenching the random sequence library to induce the formation of secondary or tertiary structures;
b) preparing a plurality of magnetic particles conjugated with negative samples (j) (NS-MPs₍ⱼ₎), wherein the magnetic particles (MPs) are nanoparticles (MNPs) or microparticles (MMPs), and wherein **j** is a variable integer with a value from 1 to **J,** and each type of **J** types of negative sample (**j**) is a different type of negative sample from the other;
c) incubating the random sequence library, from step a) if **j** equals one or from step d) of the preceding round if **j** is greater than one, with NS-MPs₍ⱼ₎ from step b) in a first PBS buffer to allow oligonucleotides to bind to the NS-MPs₍ⱼ₎, wherein the random sequence library is incubated with the NS- MPs₍ⱼ₎ one by one, or the random sequence library is incubated with all of the different NS-MPs₍ⱼ₎ in one batch;
d) removing oligonucleotides bound to the NS-MPs₍ⱼ₎ by performing a first magnetic separation using a magnetic stand and collecting a supernatant containing oligonucleotides not bound to the NS-MPs₍ⱼ₎ for step e) if **j** equals **J** or as a random sequence library for step c) of a following round if **j** is less than **J,** wherein the process steps c) and d) are performed **J** times if in step c) the random sequence library is incubated with the NS-MPs₍ⱼ₎ one by one and **j** increases with an increment of one for a following round during repetition, or if in step c) the random sequence library is incubated with all of the different NS-MPs₍ⱼ₎ in one batch, the process steps c) and d) are performed once;
e) preparing a plurality of magnetic particles conjugated with positive samples (**i**) (PS-MPs₍ᵢ₎) and incubating the supernatant containing oligonucleotides, from step d) if **i** equals one or from step i) of the preceding round if **i** is greater than one, with the PS-MPs(i) to form a bound mixture containing oligonucleotides bound to the PS-MPs₍ᵢ₎, wherein the magnetic particles (MPs) are MNPs or MMPs, each type of **I** types of the positive sample (i) is a different type of positive sample from the other and **i** is a variable integer with a value from 1 to **I;**
f) collecting the bound mixture containing oligonucleotides bound to the PS-MPs₍ᵢ₎ by performing a second magnetic separation using the magnetic stand, removing a supernatant containing oligonucleotides not bound to the PS-MPs₍ᵢ₎, and redispersing the collected bound mixture containing oligonucleotides bound to the PS-MPs₍ᵢ₎ in a second PBS buffer;
g) subjecting the redispersed bound mixture obtained in step f) to a MARAS at a first oscillating magnetic field with a lower-bound frequency f_{L} and/or a lower-bound strength H_{L} to detach oligonucleotides with a binding affinity lower than a first binding affinity toward the PS-MPs(i) from the PS-MPs₍ᵢ₎, and then removing a supernatant containing the oligonucleotides detached from the PS-MPs₍ᵢ₎ and collecting a bound mixture containing oligonucleotides bound to the PS-MPs₍ᵢ₎ by performing a third magnetic separation using the magnetic stand;
h) redispersing the collected bound mixture containing oligonucleotides bound to the PS-MPs₍ᵢ₎ obtained in step g) in a third PBS buffer;
i) subjecting the redispersed bound mixture obtained in step h) to a MARAS at a second oscillating magnetic field with an upper-bound frequency f_{U} and/or an upper-bound strength H_{U} to detach oligonucleotides with a binding affinity lower than a second binding affinity toward the PS-MPs₍ᵢ₎ from the PS-MPs₍ᵢ₎, and then collecting a supernatant containing oligonucleotides with a binding affinity lower than the second binding affinity toward the PS-MPs₍ᵢ₎ for step j) if **i** equals **I** or for step e) of a following round if **i** is less than **I** and removing a remaining bound mixture containing oligonucleotides bound to the PS-MPs₍ᵢ₎ with a binding affinity higher than the second binding affinity toward the PS-MPs₍ᵢ₎ by performing a fourth magnetic separation using the magnetic stand, wherein f_{L} ≤ f_{U} and/or H_{L} ≤ H_{U} and excludes using f_{L} = f_{U} and H_{L} = H_{U} simultaneously,
or eluting oligonucleotides with a binding affinity higher than the first binding affinity toward the PS-MPs₍ᵢ₎ from the PS-MPs₍ᵢ₎ of the redispersed bound mixture obtained in step h) and collecting a supernatant containing oligonucleotides with a binding affinity higher than the first binding affinity toward the PS-MPs₍ᵢ₎ for step j) if **i** equals **I** or for step e) of a following round if **i** is less than **I** by performing a fifth magnetic separation using the magnetic stand,
wherein the process steps e)-i) are repeated as one round for **I** times and **i** increases with an increment of one for a following round during repetition, or if the composition of the positive sample is fixed and known, the process steps e)-i) are performed once, and wherein the second binding affinity is higher than the first bind affinity; and
j) obtaining oligonucleotides as aptamers capable of distinguishing the positive samples and the negative samples.

2. The method of claim 1, wherein step b) comprises:
providing **J** types of negative samples (**j**) and separately conjugating the **J** types of negative samples (**j**) with a plurality of magnetic particles to form **J** types of NS-MPs₍ⱼ₎; and
step e) comprises:
providing **I** types of positive samples (**i**) and separately conjugating the **I** types of positive samples (**i**) with a plurality of magnetic particles to form **I** types of PS-MPs₍ᵢ₎.

3. The method of claim 2, wherein separately conjugating the **J** types of negative samples (**j**) or the **I** types of positive samples (**i**) with the magnetic particles comprises joining the **J** types of negative samples (**j**) or the **I** types of positive samples (**i**) to the magnetic particles through joining pairs respectively attached to the magnetic particles and the **J** types of negative samples (**j**) or the **I** types of positive samples (**i**).

4. The method of claim 3, wherein the joining pairs are constituted by streptavidin and biotin, and wherein the streptavidin binds with the magnetic particles and the biotin binds with the **J** types of negative samples (**j**) or the **I** types of positive samples (**i**).

5. The method of claim 1, wherein step g) subjecting the redispersed bound mixture to a MARAS at a first oscillating magnetic field with a lower-bound frequency f_{L} and/or a lower-bound strength H_{L} comprises performing a MARAS by applying a first rotating magnetic field or a first alternating magnetic field with the lower-bound frequency f_{L} and/or the lower-bound strength H_{L}.

6. The method of claim 1, wherein step i) subjecting the redispersed bound mixture to a MARAS at a second oscillating magnetic field with an upper-bound frequency fu and/or an upper-bound strength H_{U} comprises performing a MARAS by applying a second rotating magnetic field or a second alternating magnetic field with the upper-bound frequency f_{U} and/or the upper-bound strength H_{U}.

7. A method of purifying at least one type of target biomolecules from positive samples using aptamers which are capable of distinguishing the positive samples from negative samples and conjugating with the at least one type of target biomolecules in the positive samples, comprising:
a) providing the aptamers, wherein the aptamers are dispersed in a first PBS buffer, and heated and quenched to induce the formation of secondary or tertiary structures;
b) providing **N** positive samples (**n**), wherein each positive sample contains the at least one type of target biomolecules and **n** is a variable integer with a value from 1 to **N;**
c) preparing a plurality of magnetic particles conjugated with the aptamers from step a) to form an aptamer magnetic particle bound complex as a purification reagent for purifying the at least one type of target biomolecules from positive samples (**n**), wherein the magnetic particles (MPs) are nanoparticles (MNPs) or microparticles (MMPs);
d) incubating the purification reagent, from step c) if **n** equals one or from step i) of the preceding round if **n** is greater than one, with a positive sample (**n**) from step b) to form a magnetic particle-aptamer-biomolecule bound mixture during the purification process of the at least one type of target biomolecules;
e) subjecting the magnetic particle-aptamer-biomolecule bound mixture from step d) to an initial (0^{th}) oscillating magnetic field with an initial field frequency f₀ and/or an initial field strength H₀ in order that biomolecules having a binding affinity lower than an initial (0^{th}) binding affinity, including nonspecific and low binding affinity, bound to the aptamers, detach from the magnetic particle-aptamer-biomolecule bound mixture;
f) collecting a remaining magnetic particle-aptamer-biomolecule bound mixture in step e), removing a supernatant containing the biomolecules detached from the magnetic particle-aptamer-biomolecule bound mixture or unbound biomolecules by performing a first magnetic separation using the magnetic stand, and redispersing the collected magnetic particle-aptamer-biomolecule bound mixture in a second PBS buffer;
g) subjecting the redispersed magnetic particle-aptamer-biomolecule bound mixture, from step f) if **m** equals one or from step h) of the preceding round if **m** is greater than one, to a m^{th} oscillating magnetic field with a m^{th} field frequency fₘ and/or a m^{th} field strength Hₘ in order that biomolecules having a binding affinity higher than a (m-1)^{th} binding affinity and lower than a m^{th} binding affinity bound to the aptamers detach from the magnetic particle-aptamer-biomolecule bound mixture, wherein the (m-1)^{th} binding affinity is lower than the m^{th} binding affinity, and **m** is a variable integer with a value from 1 to **M** ;
h) collecting a remaining magnetic particle-aptamer-biomolecule bound mixture, retaining a supernatant containing the biomolecules detached from the magnetic particle-aptamer-biomolecule bound mixture in step g) as a separated component (**m**) for step j) by performing a second magnetic separation using the magnetic stand, and redispersing the collected magnetic particle-aptamer-biomolecule bound mixture in a third PBS buffer for step i) if **m** equals **M** or for step g) of the following round if **m** is less than **M,**
wherein the process steps g)-h) are repeated **M** times and **m** increases with an increment of one for a following round during repetition or if a suitable oscillating magnetic field range (f₍ₘ₋₁₎, H₍ₘ₋₁₎, f₍ₘ₎, and H₍ₘ₎) for a specific type of target biomolecules is known, the process steps g)-h) are performed once, until all of the biomolecules in the magnetic particle-aptamer-biomolecule bound mixture are completely detached from the aptamers and the magnetic particle-aptamer-biomolecule bound mixture reverts to an aptamer magnetic particle bound complex, and wherein f₍ₘ₋₁₎ ≤ f₍ₘ₎ and/or H₍ₘ₋₁₎ ≤ H₍ₘ₎ and excludes using f₍ₘ₎ = f₍ₘ₋₁₎ and H₍ₘ₎ = H₍ₘ₋₁₎ simultaneously;
i) using the collected and redispersed aptamer magnetic particle bound complex from step h) as a purification reagent or re-preparing a new purification reagent according to the method of step c) for the purification of the at least one type of target biomolecules from a next positive sample (**n**), and repeating the process steps d)-i) **N** times, **n** increases with an increment of one for a following round during repetition, and the applied oscillating magnetic field conditions remain unchanged for each repeated round; and
j) collecting **M** types of the corresponding separated component (**m**) obtained from step h) of sequentially performing **N** repetition of the process steps d)-i) to obtain **M** types of purified target biomolecules.

8. The method of claim 7, wherein step c) preparing a plurality of magnetic particles conjugated with the aptamers to form an aptamer magnetic particle bound complex comprises:
providing the aptamers and conjugating the aptamers with the magnetic particles to form the aptamer magnetic particle bound complex through joining pairs, and wherein the joining pairs are constituted by streptavidin and biotin, and the streptavidin binds with the magnetic particles and the biotin binds with the aptamers.

9. The method of claim 7, wherein step e) subjecting the magnetic particle-aptamer-biomolecule bound mixture to an initial (0^{th}) oscillating magnetic field with an initial field frequency fo and/or an initial field strength Ho comprises applying an initial (0^{th}) rotating magnetic field or an initial (0^{th}) alternating magnetic field with the initial field frequency f₀ and/or the initial field strength H₀ for performing a magnetically-assisted removal of the biomolecules bound to the aptamers having a binding affinity lower than the initial binding affinity, including nonspecific and low binding affinity, from the magnetic particle-aptamer-biomolecule bound mixture.

10. The method of claim 7, wherein step g) subjecting the redispersed magnetic particle-aptamer-biomolecule bound mixture to a m^{th} oscillating magnetic field with a m^{th} field frequency fₘ and/or a m^{th} field strength Hₘ comprises applying a m^{th} rotating magnetic field or a m^{th} alternating magnetic field with the m^{th} field frequency fₘ and/or the m^{th} field strength Hₘ for performing a magnetically-assisted detachment of the biomolecules bound to the aptamers having a binding affinity higher than the (m-1)^{th} binding affinity and lower than the m^{th} binding affinity from the magnetic particle-aptamer-biomolecule bound mixture.

11. A method of purifying at least one type of target biomolecules from positive samples using ligands which are capable of distinguishing positive samples from negative samples and conjugating with the at least one type of target biomolecules in the positive samples, comprising:
a) providing the ligands and dispersing the ligands in a first PBS buffer as a purification reagent for purifying the at least one type of target biomolecules from positive samples;
b) providing **N** positive sample (**n**), wherein each positive sample (**n**) contains the at least one type of target biomolecules and **n** is a variable integer with a value from 1 to **N;**
c) incubating a positive sample (**n**) from step b) with the purification reagent from step a) if **n** equals one or from step g) of the preceding round if **n** is greater than one, to form a ligand-biomolecule bound mixture through at least one type of joining pairs of the ligands and the at least one type of target biomolecules in the positive sample (**n**), during the purification process;
d) subjecting the ligand-biomolecule bound mixture from step c) to a virtual filter capable of selecting a binding affinity of the at least one type of joining pairs to remove unbound and nonspecifically-bound biomolecules toward the ligands with the joining pairs having a binding affinity lower than a 0^{th} binding affinity from the ligand-biomolecule bound mixture, and collect a remaining ligand-biomolecule bound mixture, wherein the virtual filter has an ability of changing the selectivity of the binding affinity range of the at least one type of joining pairs;
e) subjecting the ligand-biomolecule bound mixture, from step d) if **m** equals one or from step f) of the preceding round if **m** is greater than one, to a virtual filter with a selected m^{th} binding affinity higher than a (m-1)^{th} binding affinity to separate and collect a m^{th} type of target biomolecules for step h) from the ligand-biomolecule bound mixture and retain a remaining ligand-biomolecule bound mixture for step f), wherein the joining pairs formed by the m^{th} type of target biomolecules and the ligands have a binding affinity higher than the (m-1)^{th} binding affinity and lower than the m^{th} binding affinity and the (m-1)^{th} binding affinity is lower than the m^{th} binding affinity;
f) increasing gradually the binding affinity, selected by the virtual filter, of the at least one type of joining pairs formed by the ligands and the at least one type of target biomolecules in the ligand-biomolecule bound mixture in step e), and repeating the process steps e) to f) **M** rounds and **m** increases with an increment of one for a following round during repetition, or if a suitable range of the binding affinity of the joining pairs between the ligands and a specific type of target biomolecules in the ligand-biomolecule bound mixture is known, then the process steps e) to f) are performed once by using a virtual filter with the suitable range of the binding affinity of the joining pairs, until all of biomolecules in the ligand-biomolecule bound mixture are completely separated and the ligand-biomolecule bound mixture reverts to ligands;
g) redispersing the ligands from step f) in the second PBS buffer as a purification reagent or re-preparing a purification reagent according to the method of step a) for the purification of the at least one type of target biomolecules from a next positive sample (**n**), and repeating the process steps c) to g) **N** rounds and **n** increases with an increment of one for a following round during repetition, and the conditions of the virtual filter used in the purification of each positive sample (**n**) remain unchanged; and
h) collecting **M** types of the corresponding m^{th} type of target biomolecules obtained from step e) of sequentially performing the process steps c) to g) **N** rounds to obtain **M** types of purified target biomolecules.

12. The method of claim 11, wherein step c) a ligand-biomolecule bound mixture comprises forming the at least one type of the joining pairs by the ligands and the at least one type of target biomolecules, and wherein the binding affinity of joining pairs formed by the ligands and each type of the at least one type of target biomolecules is different from the other.

13. The method of claim 12, wherein the at least one type of joining pairs comprises the following pairs: antibody-antigen, DNA/RNA aptamer-captured biomolecule, and ssDNA to its complemental strand.

14. The method of claim 11, wherein step d) subjecting the ligand-biomolecule bound mixture to a virtual filter capable of selecting a binding affinity range of the at least one type of joining pairs, and wherein the selection mechanism of the virtual filter comprises applying a mechanical force, a hydrodynamic force, a centrifugal force, an electromagnetic force, or any combination thereof to the ligand-biomolecule bound mixture.

15. The method of claim 11, wherein using the ligands as a purification reagent for the purification method of the at least one type of target biomolecules from the positive samples comprises conjugating the ligands to a fixed surface for a solid-support of the purification method of the at least one type of target biomolecule from the positive samples.

16. The method of claim 11, wherein using the ligands as a purification reagent for the purification method of the at least one type of target biomolecules from the positive samples comprises providing a plurality of magnetic particles or dielectric particles and conjugating the ligands with the magnetic particles or dielectric particles suspended in an aqueous solution for the purification method of the at least one type of target biomolecules from the positive samples, and wherein the magnetic particles or dielectric particles are nanoparticles or microparticles.

17. The method of claim 11, wherein after conjugating the ligands with the magnetic particles or dielectric particles suspended in an aqueous solution for the purification method of the at least one type of target biomolecules from the positive samples comprises collecting the magnetic particles or dielectric particles conjugated with the ligands by a collecting means.

18. The method of claim 17, wherein the collecting means comprises geometric trapping, or capturing by a magnetic gradient field or an electric gradient field.
